# EUROPEAN PATENT SPECIFICATION

(11) **EP 0 742 831 B1**
(45) Date of publication and mention of the grant of the patent: **29.09.2004**
(21) Application number: 95911583.3
(22) Date of filing: 27.01.1995
(51) Int. Cl.: C12N 15/63, C12N 15/85, C12N 15/90, C12N 5/16

(54) **TRANSGENIC NON-HUMAN MAMMALS WITH PROGRESSIVE NEUROLOGIC DISEASE**
NICHT HUMANE, TRANSGENISCHE SÄUGETIERE MIT SICH ENTWICKELNDER NEUROLOGISCHER KRANKHEIT
MAMMIFERES NON HUMAINS TRANSGENIQUES ATTEINTS DE MALADIES NEUROLOGIQUES PROGRESSIVES

(30) Priority: 27.01.1994 US 189064
(43) Date of publication of application: 20.11.1996
(73) Proprietor: REGENTS OF THE UNIVERSITY OF MINNESOTA, Minneapolis, Minnesota 55455 (US); THE JOHNS HOPKINS UNIVERSITY, Baltimore, Maryland 21205 (US)
(72) Inventor: HSIAO, Karen, North Oaks, Minneapolis, MN 55127 (US); BORCHELT, David R., Baltimore, MD 21286 (US); SISODIA, Sangra M., Baltimore, MD 21212 (US)
(74) Representative: Goldin, Douglas Michael
(86) International application number: PCT/US1995/001088
(87) International publication number: WO 1995/020666

(56) References cited:
- WO-A-91/19810
- Neurology, 1994, 44 (4 Suppl. 2), A372 46th Annual Meeting of the American Academy of Neurology, Washington, D.C. USA May 1-7, 1994
- Neurobiology of Aging, 1994, 15 (suppl. 1) S71. 4th International Conference on Alzh- eimer's Disease and related disorders, Minneapolis, Minn., USA, July 29-August 3,
- Society for Neuroscience Abstracts, 1994, 20, Abstract 271.9
- CELL, vol. 46, 1986 pages 417-428, K. BASLER ET AL. 'Scrapie and cellular PrP isoforms are encoded by the same chromosomal gene'
- NATURE GENETICS, vol. 5, no. 1, 1993 pages 21-30, B.T. LAMB ET AL. 'Introduction and expression of the 400kb precursor amyloid protein gene in transgenic mice'
- NATURE, vol. 352, 1991 pages 239-241, D. QUON ET AL. 'Formation of beta-amyloid protein deposits in brains of transgenic mice'

## Description

### Introduction

### Technical Field:

The invention relates to transgenic non-human animals with a progressive degenerative neurologic disease.

### Background:

The term degenerative as applied to diseases of the nervous system is used to designate a group of disorders in which there is gradual, generally relentlessly progressing wasting away of structural elements of the nervous system; many of the conditions so designated depend upon abnormal genetic factors. The degenerative diseases manifest themselves by a number of syndromes distinguished by their clinical and pathological features. Nevertheless, there are certain aspects common to all. These aspects include having a gradually progressive course of onset, bilaterally symmetric distribution of the changes brought about by the disease, and in many cases, the almost selective involvement of anatomically or physiologically related systems of neurons. Typically the pathologic process is one of slow invaluation of nerve cell bodies or their prolongations as nerve-fibers.

Amongst the degenerative diseases of the nervous system are syndromes in which the outstanding feature is progressive dementia; the syndromes in this group include senile dementia and Alzheimer's disease. Senile dementia is a fairly frequent condition of old age. Alzheimer's disease is a pathologically identical, but much more infrequent, progressive dementia which comes on well before the senile period. The distinction between the two conditions is purely clinical; pathologically they differ only in that the characteristic abnormalities tend to be more sever and widespread in cases of Alzheimer's disease and to begin at an earlier age than at the senile period.

Alzheimer's disease (AD) shows a slowly progressive mental deterioration with failure of memory, disorientation and confusion leading to profound dementia. The disease predominantly involves limbic and cortical regions of the brain. There are several histologic features, but two are striking. First, argyrophilic plaques comprised of the amyloidogenic Aβ fragment of amyloid precursor protein (APP) are scattered throughout the cerebral cortex and hippocampus. Second, neurofibrillary tangles are found in pyramidal neurons predominantly located in the neocortex, hippocampus, and nucleus basalts of Meynert. There are other changes, also. Granulovacuolar degeneration in the pyramidal cells of the hippocampus has been considered by some to be more specific for AD than plaques or neurofibrillary tangles. Finally, there is neuronal loss and gliosis in the cortex and hippocampus.

There are patients with dementia who lack the pathologic features of AD (and by definition have a different disease), and conversely, there are individuals with many of the pathologic features of AD who were not demented prior to death. The diagnosis of AD requires that both the clinical and the pathological features characteristic for the disease be present in the patient; the diagnosis cannot be made with certainty from either clinical or pathological features alone. Whether neural dysfunction and clinical abnormalities precede the development of these pathologic features, particularly the amyloid plaques and neurofibrillary tangles, is unknown.

The clinical manifestations of AD predict the regions of affected brain structures in the forebrain, including the cerebral cortex, hippocampus, amygdala, and parahippocampal gyri. These regions are known as the cortico-limbic areas of the brain. The hindbrain is spared, including the cerebellum, the pontine and the medullary nuclei. Within the cerebral neocortex, the primary cortical area is relatively spared, which corresponds to the relative clinical sparing of basic motor and sensory cortical functions.

Research into progressive neurologic disorders such as AD has been seriously impeded by the lack of easily accessible animal models. Some aspects of the neuropathology of aged primates are similar to human AD (Price, et al., (1992) *J. Neurobiol*. 23:1277-1294). Aged primates develop amyloid plaques and forme fruste neurofibrillary tangles. No other animals studied develop disease resembling AD as closely as aged primates. However, aged primates are impractical to study in large numbers; their use raises both moral and economic issues.

Transgenic mice harboring APP transgenes have been described; however, the reported transgene product expression falls considerably short of endogenous levels of APP (total APP levels in the other transgenic mice have not exceeded 150% of endogenous levels), and fails to generate a disease phenotype with a progressive neurobehavioral disorder accompanied by pathology in the cortico-limbic regions of the brain. In these other transgenic mice, there have been no signs of a progressive neurologic disorder or of neuropathologic changes in the brain which may be regarded as evidence of a true neurologic disease. Missense point mutations in the gene coding for the amyloid precursor proteins have been linked to familial AD. However, despite the discovery of disease associated mutations in APP, most published attempts to create transgenic animals with AD have involved only wild-type APP transgenes in mice (Kawabata et al., (1991) *Nature* 354, 476-478; Quon et al., (1991); *Nature* 352, 239-41.; Wirak et al., (1991) *Science* 253, 323-325; Kammesheidt et al., (1992) *Proc Natl Acad Sci U.S.A.* 89, 10857-61; Lamb et al., (1993) *Nature Genetics* 5, 22-30.) Unfortunately, several of the published studies purporting pathology have been confounded by inadequate documentation of transgene product expression and/or misinterpretation of pathology. Two have been retracted (Kawabata, et al., 1991; Wirak, et al., 1991).

Previous efforts to create a model of AD in transgenic mice have been discouraging. In most cases, transgene product expression comparable to or exceeding endogenous levels of APP was not achieved and the transgenes did not encode mutated APP. PCT/US92/11276 reports methods for using mutant genes. In some cases, the entire APP gene was not expressed, just the carboxyl terminus (Kammesheidt, et al., (1992) *Proc Natl Acad Sci U.S.A.* 89, 10857-61); this may overlook any biologic effects that the rest of the APP molecule may exert in AD.

Preamyloid APP plaques have been observed in some transgenic mice. However, preamyloid APP plaques are not necessarily indicative of a disease, since they are routinely observed in human brain regions, such as the cerebellum, which are devoid of other signs of pathology or clinical manifestations. Increased APP immunoreactivity located within vesicular structures in hippocampal neurons of transgenic mice has been reported, but the significance of this immunoreactivity is unclear since the mice exhibited neither a progressive neurobehavioral disorder nor evidence of true neuropathology.

In general, the ceaselessly progressive course of the neurodegenerative disease is uninfluenced by current treatment modalities. It therefore is of interest to develop a transgenic nonhuman mammalian model for degenerative neurologic diseases such as senile dementia and AD wherein the animal develops a progressive degenerative neurologic disease of the cortico-limbic brain resembling the disease, both clinically and pathologically (e.g. the gliosis and the specific brain regions affected). It also is desirable that the animal develops neurologic disease within a fairly short period of time from birth, facilitating the analysis of multigenerational pedigrees. The model can be used to study the pathogenesis and treatment of degenerative neurologic diseases since there is a distinct and robust clinical and pathologic phenotype to examine and score.

### Relevant Literature:

Quon et al. (1991) *Nature* 352:239 describe transgenic mice containing human amyloid precursor protein genes. Lamb et al. (1993) *Nature Genetics* 5:22 describe transgenic mice in which the amount of amyloid precursor protein expressed is approximately 50% over endogenous levels. PCT application US 92/11276 discloses methods for constructing transgenic mice and rats which would express, under various promoters, three forms of the B-amyloid precursor protein (APP), APP 69S' APP 751, and APP 770. No data are provided in the specification as to whether expression is obtained in vivo using these methods.

Other transgenic mouse studies of Alzheimer amyloid precursor (APP) protein include the following. (Greenberg, B.D. (1993) Abstract 421.12, *Society for Neuroscience Abstracts* 19:1035.) The APP protein gene was expressed using MAPP and mMt-I promoters. Schwartz, et al. ((1993) Abstract 421.13, *Society for Neuroscience Abstracts*, 19:1035) disclose neuron-specific expression of human beta-amyloid precursor protein (APP) in transgenic mice. Savage, et al. ((1993) Abstract 421.14 *Society for Neuroscience Abstructs* 19:1035) disclose 5 human amyloid precursor protein expression in transgenic mice as a model of Alzheimer's disease: search for pathology. Lieberburg, I. ((1993) Abstract 421.15, *Society for Neuroscience Abstracts* 19:1035) disclose expression of human protein in transgenic mice using the NSE promotor. Fukuchi, K. et al. ((1993) Abstract 421.16, *Society for Neuroscience Abstracts* 19:1035) disclose intestinal beta-amyloidosis in transgenic mice. A chicken beta-actin promotor and CMV enhancer were used for expressing the APP protein gene.

Wagner et al. ((1981) *Proc*. *Nat. Acad. Sci. U.S.A.* 78:5016) describe transgenic mice containing human globin genes. Scott et al. ((1989) *Cell* 59: 847) describe transgenic mice containing hamster prion protein genes. Hsiao et al. ((1990) *Science* 250:1587) describe transgenic mice containing mutant human prion protein genes. Hsiao disclosed a model for Gerstmann-Straussler-Scheinker disease (GSS), a rare neurodegenerative disease caused by mutations in the prion protein (PrP) gene, in transgenic mice in which levels of mutant transgene product exceeding endogenous levels were needed to generate a clinical and pathological phenotype (Hsiao et al., (1990) *Science* 250:1587-1590); Hsiao, et al., (1994) *Proc. Natl*. *Acad*. *Sci. USA,* 91:9126-9130).

### SUMMARY OF THE INVENTION

A transgenic non-human animal model for progressive neurologic disease is provided, together with methods and compositions for preparation of the animal model and methods for using it. The non-human mammals are obtained by the steps of introducing multiple copies of an expression cassette into the non-human mammal at an embryonic stage, and developing the embryo to term in a pseudo-pregnant foster female. The expression cassette comprises an amyloid precursor protein coding sequence operably joined to regulatory sequences for expression of the coding sequence in neurologic tissues at a level at least two to four-fold that of endogenous levels of wildtype amyloid precursor protein. The resulting transgenic non-human mammals develop progressive neurologic disease in the cortico-limbic areas of the brain. The transgenic non-human animals find use for example in screening protocols for treatment and prevention of progressive neurologic diseases.

### DRAWINGS

Figure 1 is a diagrammatic representation of a HuAPP cDNA sequence.
Figure 2 is a diagrammatic representation of different APP sequences which can be expressed in transgenic animals (not exhaustive).
Figure 3 is a diagrammatic representation of a hamster PrP cosmid vector with a tetracycline-resistance sequence flanked by SalI sites replacing the PrP coding sequence.
Figures 4 and 5 are diagrammatic representations of a hamster PrP cosmid vector fused with HuAPP sequences modified for strong translation initiation as illustrated in Figures 6 and 7.
Figures 6 and 7 are diagrammatic representations of HuAPP sequences modified for strong translation initiation and flanking SalI restriction sites.
Figure 8 is a diagrammatic representation of PCR primers which can be used to detect transgenes.
Figure 9 shows age-related CNS dysfunction in transgenic and non-transgenic FVB mice. In two lines of Tg mice, Tg(HuAPP695.TRImyc) 1130H and Tg(HuAPP695.TRImyc)1118 expressing variant HuAPP at 3.6 and 1.4 times endogenous MoAPP levels, respectively, the average onset of illness was inversely related to APP levels. A subset of Tg(HuAPP695.WTmyc)1874 mice and non-Tg mice developed clinical and pathological abnormalities similar to those in affected Tg mice, but with significantly lower penetrance at any given age.

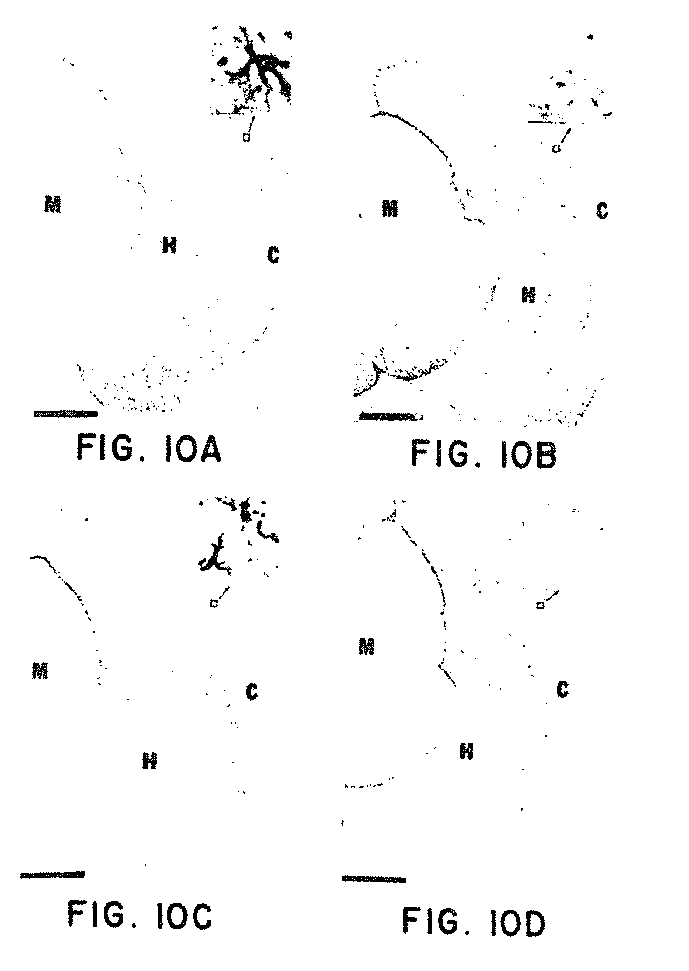

Figure 10 shows cortico-limbic hypertrophic astrocytic gliosis in transgenic and non-transgenic FVB mice exhibiting behavioral abnormalities. Coronal sections of cortico-limbic and brainstem structures reacted with antibody to GFAP show hypertrophic gliosis in cortico-limbic areas of animals exhibiting behavioral abnormalities. Figure 10A, Tg(HuAPP695.TRImyc)1118-334 exhibiting behavioral abnormalities (agitation and low corner index scores) at 144 days of age, sacrificed at 206 days; Figure 10B, non-Tg littermate of Tg1118-334 without behavioral abnormalities, age 206 days; Figure 10C, non-Tg #4565 exhibiting behavioral abnormalities (inactivity and low comer index scores) at 324 days of age, sacrificed at 334 days; Figure 10D, non-Tg littermate of #4565 without behavioral abnormalities, age 334 days.

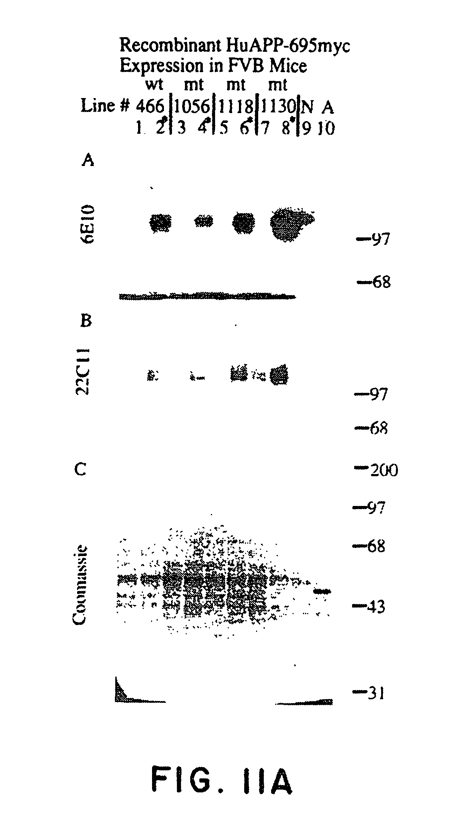

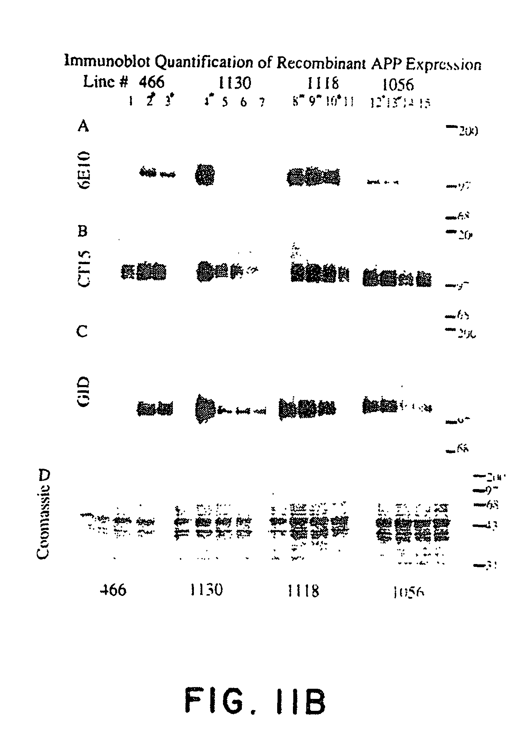

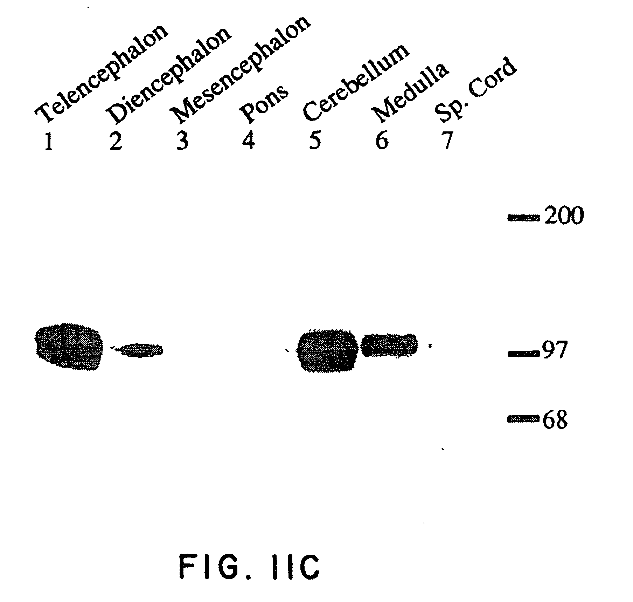

Figure 11 shows transgenic HuAPP protein expression in brain tissue. HuAPP protein expression was measured in a semi-quantitative fashion in four lines of Tg mice, Tg(HuAPP695.Wtmyc)466, Tg(HuAPP695.TRImyc)1056, Tg(HuAPP695.TRImyc)1118, Tg(HuAPP695.TRImyc)1130H, harboring 40, 7, 21 and 74 transgene copy numbers, respectively. Relative levels of transgenic compared with endogenous brain MoAPP were examined by immunoblot analysis with two polyclonal APP antisera, CT15 (Figure 11A) and anti-GID (Figure 11A), and a monoclonal antibody, 22C11 (Figure 11B). Ct15 antiserum recognized the C-terminal 15 amino acids of APP, a region in which mouse and human APP are homologous. GID antiserum recognizes an epitope 175-186 residues from the amino terminus of APP695, a region in which mouse and human APP are identical. Equivalent amounts of protein from detergent-extracted brain homogenates of non-Tg and Tg littermates were immunoblotted in parallel. Primary antibody was revealed by ¹²⁵I-protein A. For monoclonal antibodies, blots were first incubated with rabbit antiserum to mouse IgG. The amount of bound ¹²⁵I-protein A was quantified using a phosphorimager, demonstrating a direct relationship between transgene copy number and transgene product expression. To measure the level of HuAPP specifically, brain homogenates were probed with 6E10 antibody raised against residues 1-17 of human AB (Kim, et al. (1990) *Neuroscience Research Communications* 7, 113-122). Figure 11c shows the regional expression of HuAPP in the brain. The relative amount of HuAPP in 10% w/v homogenates of various tissues was specifically detected in Tg(HuAPP695.TRImyc)1130H mice using the 6E10 antibody. Equivalent amounts of protein were immunoblotted in each lane. Lanes 1, telencephalon; 2, diencephalon; 3, mesencephalon; 4, pons; 5, cerebellum; 6, mudulla; 7, spinal cord. The highest HuAPP level, in the telencephalon, was approximately twice that of the cerebellum.
Figure 12 shows the dependence of transgenic brain APP expression upon species and copy number.

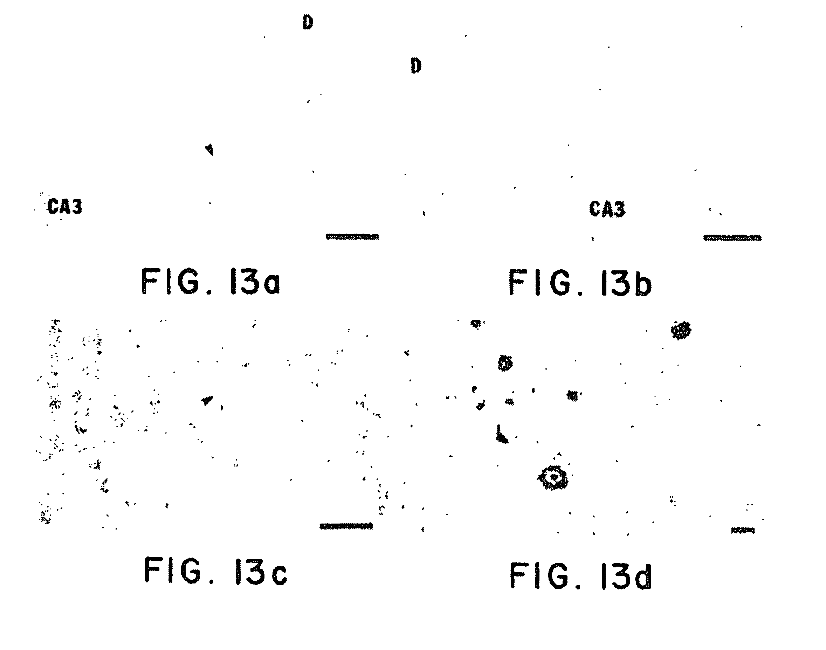

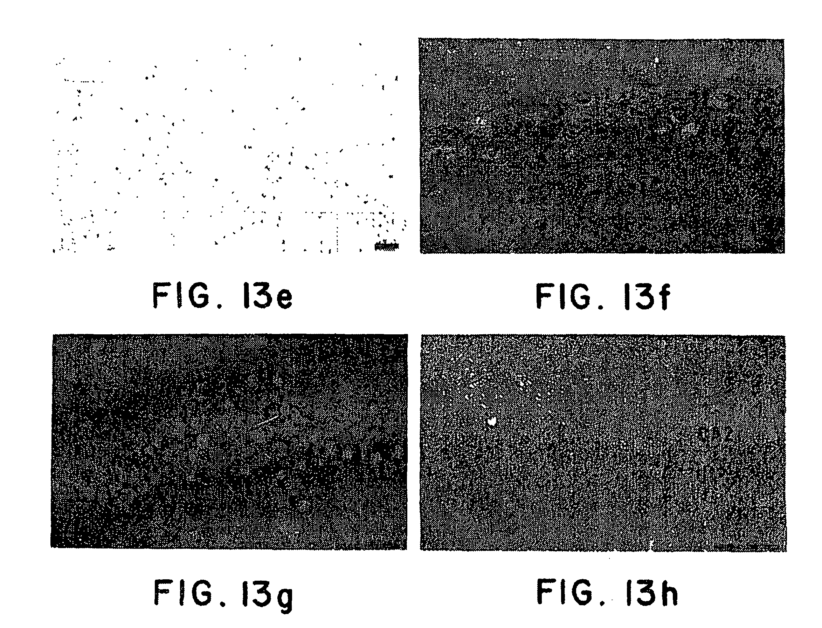

Figure 13 shows HuAPP expression in neurons of transgenic mice. Figure 13A, Tg, formic acid pretreatment, 6E10 antibody (hippocampus); Figure 13B, Non-Tg, formic acid pretreatment, 6E10 antibody (hippocampus); Figure 13C Tg, formic acid pretreatment, 6E10 antibody (cerebral cortex); Figure 13D, AD plaque, formic acid pretreatment, 6E10 antibody; Figure 13E, AD plaque, no formic acid pretreatment, 6E10 antibody; Figure 13F, AD plaque, microwave pretreatment, 8E5 antibody; Figure 13G, Tg, microwave pretreatment, 8E5 antibody (hippocampus); Figure 13H, Non-Tg, microwave pretreatment, 8E5 antibody (hippocampus).

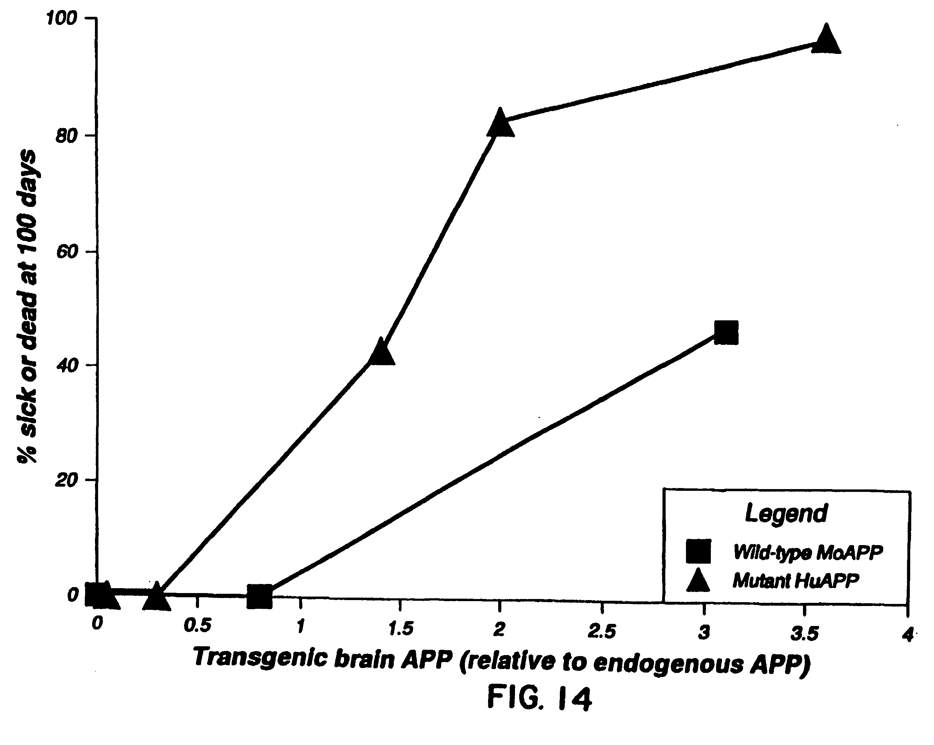

Figure 14 shows the dependence of the CNS disorder upon level of transgenic brain APP expression and APP genotype.

### DESCRIPTION OF THE PREFERRED EMBODIMENTS

The invention is directed to a transgenic nonhuman eukaryotic animal, preferably a rodent, such as a mouse, together with methods and compositions for preparing and using the animal. The animal expresses an amyloid precursor protein (APP) sequence at a level in brain tissues such that the animal develops a progressive neurologic disorder within a short period of time from birth, generally within a year from birth, preferably within 2 to 6 months, from birth. The APP protein sequence is introduced into the animal, or an ancestor of the animal, at an embryonic stage, preferably the one cell, or fertilized oocyte, stage, and generally not later than about the 8-cell stage. The zygote or embryo is then developed to term in a pseudo-pregnant foster female. The amyloid precursor protein genes are introduced into an animal embryo so as to be chromosomally incorporated in a state which results in the supra-endogenous expression of the amyloid precursor protein and the development of a progressive neurologic disease in the cortico-limbic areas of the brain, areas of the brain which are prominently affected in progressive neurologic disease states such as AD.

The present invention offers several advantages over existing models for progressive neurologic disorders such as AD. The transgenic animals express high levels of either native APP or mutant APP and develop a neurologic illness accompanied by premature death. Gliosis and intracellular APP/Aβ accretions are present in the hippocampus and cerebral cortex. The gliosis and clinical manifestations in affected transgenic animals are indicative of a true neurologic disease. The progressive aspects of the neurologic disease are characterized by diminished exploratory/locomotor behavior and diminished 2-deoxyglucose uptake/utilization and hypertrophic gliosis in the cortico-limbic regions of the brain. Further, the changes that are seen are similar to those that are seen in some aging animals.

Transgenic non-human animals of the invention are constructed using an expression cassette which includes in the 5'-3' direction of transcription, a transcriptional and translational initiation region associated with gene expression in brain tissue, DNA encoding a mutant or wild-type an APP, and a transcriptional and translational termination region functional in the host animal. One or more introns may also be present.

For expression, of particular interest are initiation regions (also sometimes referred to as "promoters") which provide for preferential or at least substantially specific expression in brain as compared to other tissue. By "at least substantially" is intended that expression in brain tissue is greater than about 10 fold that in other tissue. Within the brain, of particular interest is expression in the cortico-limbic area. The transcriptional initiation region may be endogenous to the host animal or foreign or exogenous to the host animal. By foreign is intended that the transcriptional initiation region is not found in the wild-type animal host into which the transcriptional initiation region is introduced. By endogenous, is intended sequences both indigenous (i.e. natural to) the host animal and those present in the host animal as a result of an infectious disease, e.g. viral, prion, and the like.

The promoter preferably comprises a transcriptional initiation regulatory region and translational initiation regulatory region of untranslated 5' sequences, "ribosome binding sites", responsible for binding mRNA to ribosomes and translational initiation. The transcriptional initiation regulatory region may be composed of cis-acting subdomains which activate or repress transcription in response to binding of transacting factors present in varying amounts in different cells. It is preferred that all of the transcriptional and translational functional elements of the initiation control region are derived from or obtainable from the same gene. In some embodiments, the promoter is modified by the addition of sequences, such as enhancers, or deletions of non-essential and/or undesired sequences. By "obtainable" is intended a promoter having a DNA sequence sufficiently similar to that of a native promoter to provide for the desired specificity of transcription of a DNA sequence of interest. It includes natural and synthetic sequences as well as sequences which may be a combination of synthetic and natural sequences.

Tissue-specific transcription suggests that gene regulatory proteins may be bound to enhancer sequences and other upstream promoter elements. By enhancer element ("enhancer") is intended a regulatory DNA sequence that is capable of activating transcription from a promoter linked to it with synthesis beginning at the normal RNA start site; which is capable of operating in both orientations (normal or flipped); and which is capable of functioning even when moved either upstream or downstream from the promoter. Both enhancers and other upstream promoter elements bind sequence specific DNA binding proteins that mediate their effects. To identify the exact nucleotide sequences important for the function of the enhancers(s), and other upstream elements, fragments of the untranslated 5'-region encoding a protein expressed in a tissue of interest are screened for their capacity to bind nuclear proteins and for their ability to function with a heterologous promoter. Binding experiments with nuclear proteins from brain tissue can be used to determine the presence of enhancer and silencer sequences; the protein binding studies can be used to pinpoint specific nucleotide sequences that bind to a corresponding series of gene regulatory proteins.

The activity of each enhancer and other upstream promoter elements generally is present on a segment of DNA which may contain binding sites for multiple proteins. The binding sites can generally be dissected by preparing smaller mutated versions of the enhancer sequence joined to a reporter gene whose product is easily measured. The effect of each mutation on transcription can then be tested. Alternatively, fragments of this region can be prepared. Each of the mutated versions of the enhancer sequence or the fragments can be introduced into an appropriate host cell and the efficiency of expression of a reporter gene measured. Those nucleotides required for enhancer function in this test are then identified as binding sites for specific proteins by means of gel mobility shift and DNA foot printing studies.

An alternate means of examining the capability of isolated fragments of the region upstream of the promoter to enhance expression of the reporter gene is to look for sub-domains of the upstream region that are able to enhance expression levels from a test promoter which comprises the TATA CAAT box but shows little or no detectable activity. A fragment of the 5'-region is inserted in front of the test promoter in an expression cassette, and the effect on expression of the reporter gene evaluated.

Of particular interest for brain-specific, copy number-dependent expression are regions capable of binding to nuclear proteins in the region up to about 20kb from the mRNA start site of a brain-specific protein gene. Within this region, there may be several subdomains of interest having the characteristics of brain specific enhancer elements which can be evaluated by using constructs.

A promoter from a gene expressed in brain tissue of the host animal may be employed for varying the phenotype of the host animal. The transcriptional level should be sufficient to provide an amount of RNA capable of producing in a modified animal. By "modified animal" within the subject invention is meant an animal having a detectably different phenotype from a non-transformed animal of the same species, for example one not having the transcriptional cassette including APP coding sequences in its genome. Various changes in phenotype are of interest. These changes may include progressive neurologic disease in the cortico-limbic areas of the brain expressed within a short period of the time from birth; increased levels of expression of an APP gene above endogenous expression levels and the development of a neurologic illness accompanied by premature death; gliosis and intracellular APP/Aβ accretions present in the hippocampus and cerebral cortex; progressive neurologic disease characterized by diminished exploratory/locomotor behavior and diminished 2-deoxyglucose uptake/utilization and hypertrophic gliosis in the cortico-limbic regions of the brain.

Of particular interest as a transcriptional initiation region is one from a prion protein gene which is functional in the brain of the host animal. Prion protein is implicated in the pathogenesis and transmission of Gerstmann-Straussler syndrome and in scrapie, an equivalent animal disease. Brain tissue serves as a source for nucleic acid for preparing the desired sequences. To identify a prion promoter having the desired characteristics, where a prion protein has been or is isolated, it may be partially sequenced, so that a probe may be designed for identifying mRNA specific for prion protein. Sequences which hybridize to the cDNA may then be isolated, manipulated, and the 5'untranslated region associated with the coding region isolated and used in expression constructs to identify the transcriptional activity of the 5'-untranslated region. As appropriate, sequences can be amplified using PCR procedures known to those skilled in the art. In some instances, a probe may be employed directly for screening a genomic library and identifying sequences which hybridize to the probe. The sequences will be manipulated as described above to identify untranslated region. Prion promoter sequences are described in Basler, et al. (1986), *Cell* 46:417-428 and Scott, et al. (1992) *Protein Science* 1:986-987.

A variety of other promoter sequences can be used to control expression of APP coding sequences. These include the metallothionine (MT) promoter expression from which can be regulated through modulation of zinc and glucocorticoid hormone levels (Palmiter et al., *Nature* 300, 611-615 (1982)); the rat neuron specific enolase gene promoter (Forss-Petter et al., *Neuron* 5; 197-197 (1990)); the human β actin gene promoter (Ray et al., *Genes and Development* (1991) 5:2265-2273); the human platelet derived growth factor B (PDGF-B) chain gene promoter (Sasahara et al, *Cell* (1991) 64: 217-227); the rat sodium channel gene promoter (Maue et al., *Neuron* (1990) 4:223-231); the human copper-zinc superoxide dismutase gene promoter (Ceballos-Picot et al., *Brain Res.* (1991) 552:198-214); and promoters for members of the mammalian POU-domian regulatory gene family (Xi et al., (1989) *Nature* 340:35-42). The POU-domain is the region of similarity between the four mammalian transcription factors Pit-1, Oct-1, Oct-2, and *unc*-86, and represents a portion of the DNA-binding domain. These promoters provide for expression specifically within the neurons of transgenic animals.

The termination region which is employed primarily will be one of convenience, since the termination regions appear to be relatively interchangeable. The termination region may be native with the transcriptional initiation region, may be native with the DNA sequence of interest, or may be derived from another source. Convenient termination regions are available from the prion protein gene.

The expression cassette which is used in the subject invention includes promoter and enhancer sequences from a gene which is expressed in the brain and preferably which is expressed in a manner that is related to the number of such sequences incorporated into the chromosome, namely that higher transcription occurs with a larger number of transgene copies incorporated into the chromosome, operably joined to an APP gene sequence and translational and transcriptional termination regions. Examples of promoter and enhancer sequences which are expressed in brain and which drive copy number dependant expression include the prion protein promoter, such as that described by Scott et al . Protein Science ( 1992) 1:986-987, together with sequences upstream from the promoter, because in order to obtain copy number dependant expression, it generally is necessary to include a sufficiently large region of DNA controlling transcription so that it is large enough to be relatively unaffected by position effects. As an example, for the prion protein gene from hamster, approximately 20kb of sequence upstream of the promoter can be used.

As an example of construction of a cosmid vector for use in the instant invention, components which are assembled, in the 5' to 3' direction, include promoter and enhancer sequences of the prion protein gene, the coding region of an APP gene sequence of interest and transcriptional and translational termination sequences operably attached to a cosmid vector for delivery of the DNA constructs into the pronuclei of mouse eggs for expression of an APP gene in brain tissue. The enhancer sequences may include a 20 kb region upstream of the prion protein promoter and may also include the noncoding exon 1 and the 10 kb intron downstream of exon 1 from the prion protein gene or can include the coding sequence for more than one APP protein as described in, for example, W092/11276. Using molecular genetic techniques well known in the art, the promoter/enhancer region of the prion protein gene may be isolated from a mammalian genomic cosmid clone used to create transgenic mice which express prion protein. The coding sequence of an APP gene is inserted between the promoter/enhancer region and the termination sequences at a unique restriction site or sites such that the coding sequence is translated in-frame. An APP protein in transgenic brain tissue introduced using a cosmid vector as described above may be confirmed to be at least two to four-fold that of endogenous levels.

A major obstacle to the creation of a transgenic model of AD has been the inability to overexpress transgenic APP protein in the brain of the transgenic animal. In some cases, mRNA is well expressed, but the protein is poorly expressed. This indicates that the strength of promoters used may be adequate, but that protein translation may not be optimal. Poor translation may result from a weak translation initiation sequence. Accordingly, it may be necessary to include a translation initiation sequence wherein the positions at minus three and plus four relative to the initiation codon are A and G, respectively. See Table 1 below.

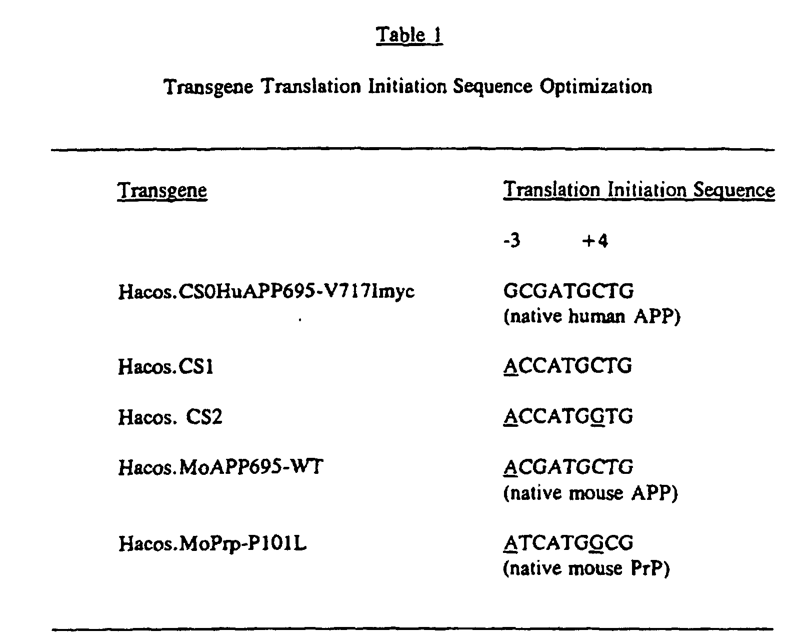

Any amyloid precursor protein sequence can be used to produce the transgenic animals of the invention. An APP protein sequence, as the term is used herein, means a sequence of the coding region of the APP gene which, when incorporated into the genome of the animal in multiple copies and expressed in the transgenic animal at supraendogenous levels, promotes a progressive neurologic disease in the transgenic animal. The neurologic disease is characterized by a neurobehavioral disorder with gliosis and diminished glucose uptake and/or utilization in cortico-limbic brain structures. The coding sequence can be from a wild-type gene, or from a gene containing one or more mutations. The coding sequence can be a natural sequence or a synthetic sequence or a combination of natural and synthetic sequences. By mutant is intended any APP which has an amino acid sequence which differs from that of the native APP and includes . substitutions, deletions, and the like. By wild type APP is intended native APP as it occurs in the relevant host animal.

Native human APP is encoded by a single 400-kb gene comprised of 18 exons on chromosome 21. Alternative mRNA splicing gives rise to three APP isoforms. Two forms, APP-751 and APP-770 contain a Kunitz-protease inhibitor (KPI) region; the third, APP-695, lacks the KPI segment. Preferred sequences are those which are disease-linked. Examples of disease-linked mutations include a mutation at APP codon 693 (of APP-770) linked to Dutch congophilic angiopathy (Levy et al., (1990) *Science* 248:1124), a mutation in APP linked to familial AD, valine-isoleucine at codon 717 (of APP770) (Goate et al., (1991) *Nature* 349: 704-706), a mutation wherein the valine at codon 717 is replaced by phenylalanine or glycine (Chartier-Harlin et al., (1991) *Nature* 353: 844-846; Murrell et al., (1991) *Science* 254: 97-99); and in one family with both congophilic angiopathy and AD, a mutation wherein alanine is replaced by glycine at codon 692 (Hendriks et al., (1992) *Nature Genetics* 1:218-221). In a Swedish kindred, a double mutation at codons 670 and 671, resulting in a substitution of the normal lysine-methionine dipeptide by asparagine-leucine was found (Mullan et al ., (1992) *Narure Genetics* 1: 345-347). APP with M67ON-K67IL is reported to be associated with increased AB 1-40 secretion (Citron *et al*. (1992) *Nature* 360: 672-674; Cai et al. (1993) *Science* 259: 514-516), while enhanced AB 1-42 production is reported for APP with the V717I mutation (Cai et al. (1993), *supra*; Suzuki *et al*. (1994) *Science* 264: 1335-1340).

Table 2, below, lists some of the known amyloid precursor protein sequences, some of which are genetically linked to familial Alzheimer's disease.

**Table 2 1**

| Examples of APP Transgenes | | | |
|---|---|---|---|
| Translation Initiation | APP ORF Species | ORF Size (codons) | Mutation |
| | | | V7171 |
| | | | V717G |
| | human, mouse | 695 & | V717F |
| CS1 or | or | 751 or | VVM717/721/7221AV |
| CS2 | human/mouse | 770 | MK670/671NL770 |
| | chimeras | | A692G |
| | | | E693Q |
| ¹The abbreviations used in Table 2 refer to the following: CS1=translation initiation sequence as represented in FIG. 6; CS2=translation initiation sequence as represented in FIG. 7; V=valine; I=isoleucine; G=glycine; F=phenylalanine; M=methionine; A=alanine; X=lysine; N=arginine; L-leucine; E=glutamate; Q=glutamine; ORF=open reading frame; numeral in the 'Mutation' column refers to the mutated codon based upon the APP-770 numbering system. | | | |

Of particular interest are novel chimeric APP genes, in which human Aβ sequences replace the Aβ region of mouse APP. A 158,5 is a 4-kDa peptide derived from APP. Examination of 5 human (Hu), mouse (Mo), and chimeric (Mo/Hu) APP processing in mouse cell lines indicates that tangible differences are evident. It appears that HuAPP matures poorly in mouse cells, relative to Mo- or combination Mo/HuAPP. However, the human Aβ sequences promote the formation of soluble Aβ peptides that are normally produced. Mo/HuAPP chimeric protein matures more efficiently than HuAPP, and generates more soluble Aβ than MoAPP.

Transgenic non-human mammals are prepared in a number of ways. A transgenic organism is one that has an extra or exogenous fragment of DNA in its genome. In order to achieve stable inheritance of the extra or exogenous DNA fragment, the integration event must occur in a cell type that can give rise to functional germ cells, either sperm or oocytes. Two animal cell types that can form germ cells and into which DNA can be introduced readily are fertilized egg cells and embryonic stem cells.

Embryonic stem (ES) cells, can be returned from *in vitro* culture to a "host" embryo where they become incorporated into the developing animal and can give rise to transgenic cells in all tissues, including germ cells. The ES cells are transfected in culture and then the mutation is transmitted into the germline by injecting the cells into an embryo. The animals carrying mutated germ cells are then bred to produce transgenic offspring.

A preferred method for making the subject transgenic non-human animals is by zygote injection. This method is described, for example, in USPN 4,736,866. The method involves injecting DNA into a fertilized egg, or zygote, and then allowing the egg to develop in a pseudo-pregnant mother. The zygote can be obtained using male and female animals of the same strain or from male and female animals of different strains. The transgenic animal that is born is called a founder, and it is bred to produce more animals with the same DNA insertion. In this method of making transgenic animals, the new DNA typically randomly integrates into the genome by a nonhomologous recombination event. One to many thousands of copies of the DNA may integrate at one site in the genome.

Generally, the DNA is injected into one of the pronuclei, usually the larger male pronucleus. The zygotes are then either transferred the same day, or cultured overnight to form 2-cell embryos and then transferred into the oviducts of pseudo-pregnant females. The animals born are screened for the presence of the desired integrated DNA. By a pseudo pregnant female is intended a female in estrous who has mated with a vasectomized male; she is competent to receive embryos but does not contain any fertilized eggs. Pseudo-pregnant females are important for making transgenic mice since they serve as the surrogate mothers for embryos that have been injected with DNA or embryonic stem cells.

Putative founders are screened for presence of the transgene in several ways. Brain APP protein and RNA expression are analyzed and the transgene copy number determined using methods known to those of skill in the art. Brain APP protein RNA expression, and transgene copy numbers are determined in weanling animals (4-5 weeks). Because the prion protein gene promoter is constitutionally active in animals of weanling age and older, it is not expected that there will be changes in levels of transgenic APP RNA expression animals beyond weanling age. APP levels can be monitored to determine whether there is a consistent variation in expression levels with age. The transgenic animals also are observed for clinical changes. Examples of neurobehavioral disorders for evaluation are poor mating response, agitation, diminished exploratory behavior in a novel setting, inactivity, seizures and premature death. For transgene copy number, sufficient copies to achieve total brain APP expression from each construct from at least twofold, preferably at least two to fourfold, that of an endogenous native gene, is preferred. This number may range from five copies to more than 60 copies, depending on the species of APP expressed and the particular disease-associated mutations in the APP gene. Sufficient copies of a transgene therefore is that number which produces expression of APP at a level which results in a progressive neurologic disorder.

It is a theory of the invention that the clinical changes observed in transgenic mice are as a result of an increase in the amount of APP which is expressed, therefore sufficient copies of an APP gene are necessary to achieve a level of expression of particular APP gene which will result in observable clinical and/or behavioral symptoms, together with a measurable biochemical change in relevant brain structures. Less desirably, only biochemical changes would be obtained. By sufficient copies is intended that the total expression level from each construct is at least two-fold, preferably at least two to four-fold, that of an endogenous native gene, or that the overall copy number is such as to achieve this relative increase. In some instances, two to four copies of the gene, especially a mutated disease-linking gene, may be sufficient to achieve a desired relative increase in APP, while in others, particularly where a native gene is used, a larger copy number may be required. In some instances a lower amount of APP may be effective in producing a progressive neurologic disorder, particularly where the mutation in the APP occurs in the Aβ region, or just upstream of the Aβ region of the gene. The number of copies of a particular gene which are sufficient to obtain the desired result can be determined empirically. As an example, the effective range of copy numbers for HuAPP695.TRImyc is approximately 20 to 75; for HuAPP695.SWE is approximately 30 to 50; and MoAPP.wg is greater than 25.

The founder animals can be used to produce stable lines of transgenic non-human animals that superexpress APP, either mutant or native APP. For ease of propagation, male founder mice are preferred. The animals are observed clinically. Analyses of transgene copy number (to exclude multiple transgene insertion sites), mRNA expression, protein expression, neuropathology, and glucose uptake in these animals are also performed. These studies provide information about the age of onset of illness, the duration of illness, the penetrance of the phenotype, the range of neuropathologic 5 findings, regional brain dysfunction, and the dependence of phenotype upon levels of protein expression.

The animals also are screened using a species appropriate neurobehavioral test. For example, studies of locomotor/exploratory behavior in mice is a standard means of assessing the neuropsychology (File and Wardill, (1975) *Psychopharmacologia* (Berl) 44:53-59; Loggi et al., (1991) *Pharmacol*. *Biochem. Behav.* 38:817-822). For example, for mice the "corner index" (CI) is used. This is a quick and simple neurobehavioral test to screen animals for evidence of brain pathology. The CI in transgenic mice which express mutant and wild-type APP is also measured. A low CI (≤4) correlates with high mutant APP transgene copy numbers, premature death, and neuropathologic findings. The CI exhibits a dosage dependent relationship to transgene copy number, which supports the validity of its use in assessing neurobehavioral signs in transgenic mice.

The neuropathology of the animals also is evaluated. Brain regions known to be affected by AD, such as those in the cortico-limbic region, are particularly reviewed for changes including APP/Aβ excretions, gliosis, and changes in glucose uptake and utilization. Immunohistologic studies of various brain regions is used to detect transgene product.

The animals used as a source of fertilized eggs cells or embryonic stem cells, the "host animal", can be any non-human animal, although generally the preferred host animal is one which lends itself to multigenerational studies. Of particular interest are rodents including mice, such as mice of the FVB strain and crossed of commercially available strains such as the (C57B6) x (SJL.F1) hybrid and the (Swiss Webster) x (C57B16/DBA-z.F1) hybrid. The latter parental line also is referred to as C57B16/D2. Other strains and cross-strains of animals can be evaluated using the techniques described herein for suitability for use as a model for progressive neurologic diseases such as AD. In some instances, however, a primate, for example a rhesus monkey may be desirable as the host animal, particularly for therapeutic testing.

The non-human animals of the invention can be used as tester animals for materials of interest, *e.g.* antioxidants such as Vitamin E or lazaroids, thought to confer protection against the development of AD. An animal is treated with the material of interest, and a reduced incidence or delayed onset of neurologic disease, as compared to untreated animals, is detected as an indication of protection. The animals further can be used as tester animals for materials of interest thought to improve or cure Alzheimer's disease. An animal with neurologic disease is treated with the material of interest, and a delayed death, or improvement in neurobehavior, gliosis, or glucose uptake/utilization, as compared to untreated animals with neurologic disease, is detected as an indication of amelioration or cure.

The non-human animals of the invention can be used to test a material or situation, e.g. oxidants or head trauma, suspected of accelerating or provoking Alzheimer's disease, by exposing the animal to the material or situation and determining neurobehavioral decline, premature death, gliosis, and diminished glucose uptake/utilization as indicators of the capacity of the test material or situation to induce Alzheimer's disease. The method further can include testing of therapeutic agents by exposing animals to a material or situation suspected of provoking Alzheimer's disease and evaluating the effect of the therapeutic agent.

Careful characterization of the transgenic animals should lead to elucidation of the pathogenesis of AD. The sequence of molecular events in mutant APP metabolism leading to disease can be studied. The animals also are useful for studying various proposed mechanisms of pathogenesis, including horizontal transmission of disease (Prusiner, et al. (1987) *Cell* 63, 673-86), oxidation and free-radical production (Blass and Gibson, (1991) *Rev*. *Neurol* (Paris) 147:513-525; Ames et al., (1993) *Proc. Nat'l*. *Acad. Sci. USA* 90:7915-7922), inflammation (McGeer et al., (1993) *Can*. *J. Neurol. Sci.* 18:376-379, Rogers et al., (1992) *Proc. Nat'l*. *Acad. Sci. USA* 89:10016-10020); neurotrophic factor deprivation (Perry, (1990) *Alzheimer's Disease and Associated Disorders* 4:1-13; Hefti and Schneider, (1991) *Clinical Neuropharmacology* 1:62-76); Koliatsoess et al., (1991) *Ann. Neurol.* 30:831-840), apolipoprotein E4 metabolism (Strittmatter et al., (1993) *Proc. Nat'l*. *Acad. Sci. U.S.A.* 90:1977-1981), and potassium channel dysfunction (Etcheberrigaray, et al., (1993) *Proc. Nat'l*. *Acad. Sci. USA* 90:8209-8213). Such knowledge would lead to better forms of treatment for neurologic disorders.

Other features and advantages of the invention will be apparent from the description of the preferred embodiments, and from the claims. The following examples are offered by way of illustration and not by way of limitation.

### EXAMPLES

### Examples 1

### PrP-HuAPP Transgene Construction.

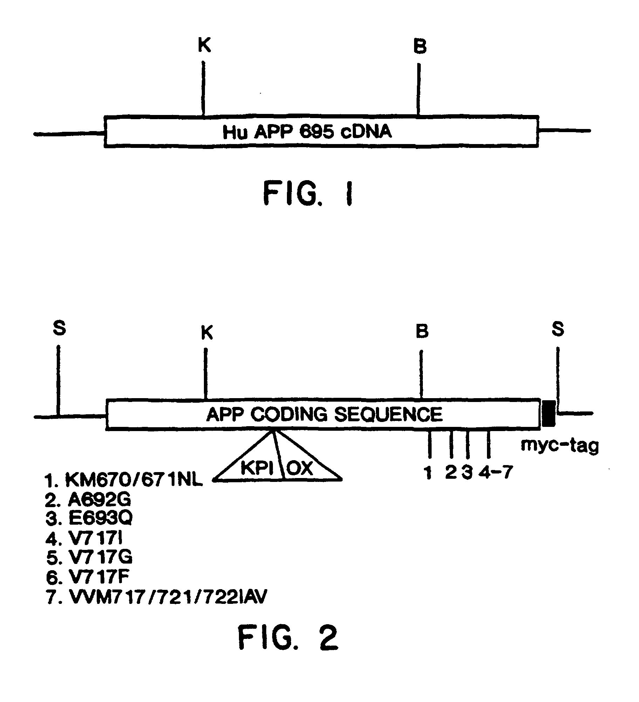

The human APP coding sequence was derived from a human cDNA (see Kang et al. (1987) *Nature* 325:733; Goldgabar et al. (1987); *Science* 235:877; Tanzi et al. (1987); *Science* 235:880; and Robakis et al. (1987) *Proc*.*Nat*.*Acad*.*Sci*. (USA) 84:4190) and is illustrated in FIG. 1. It occurs in three splice forms which are derived from a gene located on chromosome 21 as described by Kitaguchi et al. (1988) *Nature* 331:530; Tanzi et al. (1988) *Nature* 331:528; and Ponte et al. (1988) *Nature* 331:525. FIG. 2 illustrates three features which may be incorporated into amyloid precursor protein sequences to produce the transgenic animals of the invention: (1) splice form variants which result from the presence or absence of the Kunitz protease inhibitor with or without the OX region; (2) amyloid precursor protein variants containing mutations which have been linked to illness in families with Alzheimer's disease as described by Goate (1991) *Nature* 349:704; Chartier-Harlin et al. (1991) *Nature* 353:844; Murrell et al. (1991) *Science* 254:97; Hendriks et al. (1992) *Nature Genetics* 1:218; and Mullan et al. (1992) *Nature Genetics* 1:345, and families with congophilic angiopathy as described by Levy et al. (1990) *Science* 248:1124, and (3) a myc-tag at the carboxyl terminus which can be used to facilitate immunodetection of transgene products, but is preferably absent.

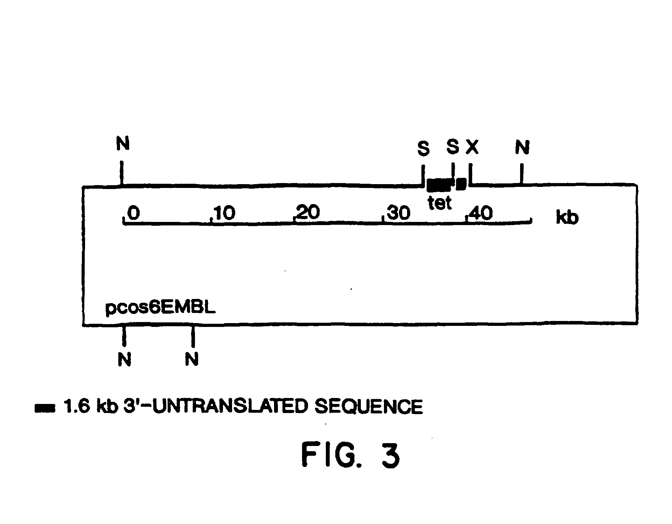

The required hamster prion protein gene functions were provided by a hamster prion protein cosmid vector in which a tetracycline-resistance sequence flanked by *Sal*I sites replaces the prion protein coding sequence, as described by Scott et al. (1992) *Protein Science* 1: 986. The hamster prion protein cosmid vector is illustrated in FIG. 3. A 1.6 KB region of DNA in the 3'-untranslated region of the prion protein gene is indicated as a useful probe for detecting transgenes made from this cosmid.

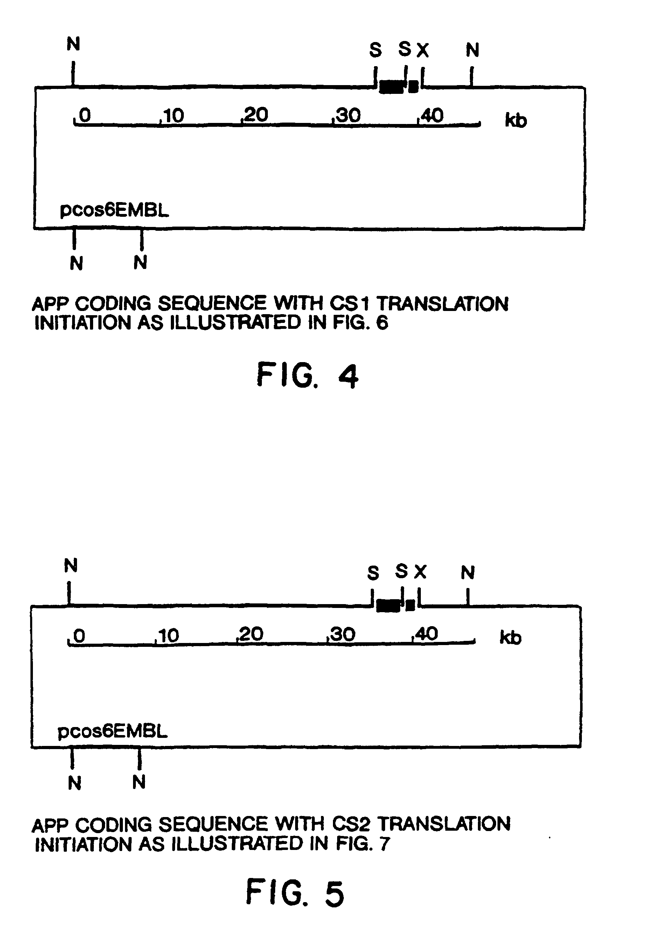

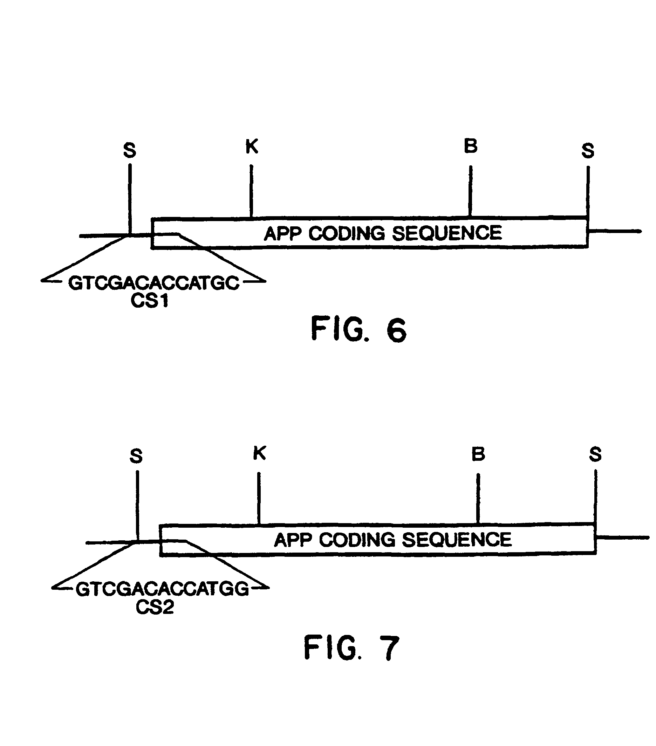

The APP sequences and cosmid were used to construct the two fusion gene constructions illustrated in FIGS. 4 and 5. The APP sequences were modified for strong translation initiation, represented by the abbreviations CS1 and CS2. The constructions were made by substituting the *Sal*I to *Kpn*I DNA sequence at the 5' end of the APP coding sequence for DNA sequences made using the polymerase chain reaction (PCR) and two sets of primers. For the CS1 APP sequence illustrated in FIG. 6, the primer set used was 5'-AAGTCGACACCATGCT GCCCGGTITGGCACT-3' and 5'-AAGGTACCTCCCAGCGCCCGAGCC-3'. For the CS2 APP sequence illustrated in FIG. 7, the primer set used was S'-AAAAAAGTCGACACCATGGTGCCCGGTTTGGCACT-3' and 5'-AAGGTACCTCCAGCGCCCGAGCC-3'.

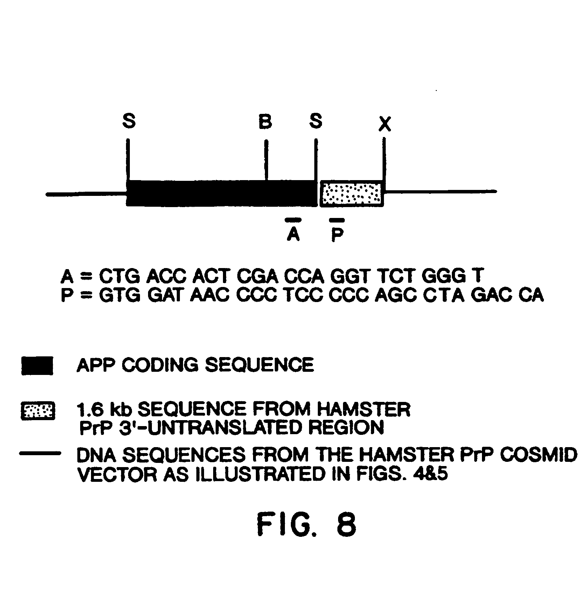

Procedures were the conventional techniques described in Maniatis et al. (1982) *Molecular Cloning: A Laboratory Manual* (Cold Spring Harbor Laboratory) and the polymerase chain reaction (PCR) described in Saiki et al. (1988) *Science* 239:487. The restriction sites shown in FIGS. 1-7 are *Sal*I *(S), Kpn*I (K), *Bgl*II (B), *Xho*I (X) and *Not*I (N). The location of the PCR oligomers used for detecting fusion constructs in animals are indicated by A and P in FIG. 8. Each PCR fragment synthesized for the constructions was sequenced. The PCR fragments selected for use in the constructions were free of unintended mutations.

The above PrP-APP cosmids were digested with *Not*I which releases the PrP-APP fusion gene from the pcos6EMBL vector illustrated in FIGS. 3-5. The PrP-APP fusion gene was isolated after size fractionation on an agarose gel and electroeluted. The PrP-APP fusion gene was further purified in a series of organic extractions, including phenol-chloroform, chloroform, and butanol, and precipitated in ammonium acetate and ethanol. Prior to embryoinjection, the PrPAPP fusion gene was dissolved in 10mM Tris-Cl (pH 8.0) to a final concentration of 3-4 µg/ml.

### Example 2

### Production of Transgenic Mice Containing PrP-HuAPP Transgene (APP Sequence VM717/721/722IAV)

Each PrP-APP fusion gene was separately injected into fertilized one-cell mouse eggs (Hogan *et al*. (1986) *Manipulating the Mouse Embyro: A Laboratory Manual*, Cold.Spring Harbor Press, N.Y.; also see USPN 4,736,866). Embryo donors and fertile studs were inbred FVB mice obtained from the National Cancer Institute (NIH); this resulted in the integration of between 1 and 128 copies of PrP-APP fusion genes into the genomes of the mice which developed to term. The injected eggs were transferred to pseudo-pregnant foster females as described in Wagner et al. (1981) *Proc. Natl*. *Acad. Sci. U.S.A. 78:5016*. Mice were housed in an environmentally controlled facility maintained on a 10 hour dark: 14 hour light cycle. The eggs in the foster females were allowed to develop to term.

### Example 3

### Analysis of VVM717/721/722IAV Transgenic Mice

At four weeks of age, each pup born was analyzed in a PCR reaction using DNA taken from the tail. In each case, tail DNA was used as a template for a PCR reaction using the probes indicated in FIG. 8. The DNA for analysis was extracted from the tail by the method described in Hanley and Merlie (1991) *Biotechniques* 10:56. 1 ml of the tail DNA preparation (approximately 1 mg of DNA) was used to amplify a transgene specific DNA fragment in a 25Z1 PCR reaction containing primers A and P as illustrated in FIG. 8.

The PCR reactions indicated that 15 founder mice had retained an injected PrP-APP fusion gene. The APP sequence in these animals contained the VVM717/721/722IAV mutation and the myc-tag, but lacked the KPI/OX regions represented in FIG. 2. To determine transgene copy number, denatured DNA in an exponentially diluted series was probed with a 1.6 kilobase (KB) radiolabelled segment of DNA from the 3'-untranslated region of the hamster PrP gene as illustrated in FIG. 3. Among the founder mice with the highest transgene copy numbers (approximately 100 or more), two founder mice failed to breed, and a third founder sired offspring, which in turn failed to breed. Thus, the 15 founder mice yielded 12 lines of transgenic offspring. A catalog of transgenic founders with APP transgenes is shown in Table III.

The founder animals were mated to uninjected animals and the DNA of the resulting 12 lines of transgenic offspring analyzed: this analysis indicated that in every case the injected genes were transmitted through the germline.

**Table III**

| Catalog of Transgenic Founders with APP Transgenes | | | | |
|---|---|---|---|---|
| Animal ID | Transgene | Transgene Copy # | Protein Level | Status |
| Tg425L | Hacos.CSOHuApp695-V717Imyc | 1 | Not detectable | Sac'd |
| Tg466M | Hacos.CSOHuApp695-WTmyc | 32-64 | 1.5-2X | Alive |
| Tg1056L | Hacos.CS1HuApp695-V717Imyc | 16 | | Alive |
| Tg1057H | Hacos.CS1HuApp695-V717Imyc | 64-128 | | Dead |
| Tg1064L | Hacos.CS1HuApp695-V717Imyc | 8 | | Alive |
| Tg1072L | Hacos.CS2HuApp695-V717Imyc | 1 | | Alive |
| Tg1073L | Hacos.CS2HuApp695-V717Imyc | 1 | | Alive |
| Tg1118M | Hacos.CS1HuApp695-V717Imyc | 32-64 | | Alive |
| Tg1119L | Hacos.CS1HuApp695-V717Imyc | 1 | | Alive |
| Tg1123L | Hacos.CS1HuApp695-V717Imyc | 1 | | Alive |
| Tg1125L | Hacos.CS1HuApp695-V717Imyc | 8-16 | | Alive |
| Tg1130H | Hacos.CS1HuApp695-V717Imyc | 64-128 | | Sick |
| Tg1135H | Hacos.CS2HuApp695-V717Imyc | 64-128 | | Dead |
| Tg1138H | Hacos.CS2HuApp695-V717Imyc | 64-128 | | Dead |
| Tg1140M | Hacos.CS2HuApp695-V717Imyc | 32-64 | | Alive |

Six founder animals harbored > 20 copies of the PrP-APP fusion genes. All six developed a neurologic disease characterized by progressively diminishing exploratory/locomotor behavior and premature death by five months of age. In contrast, none of nine founder animals harboring < 20 copies of the PrP-APP fusion genes have developed the neurologic disease within the first five months of age. The neurologic dysfunction was transmitted to succeeding generations in an autosomal dominant fashion.

Expression of the newly acquired PrP-APP fusion genes in tissues was determined by Western blot analysis using a monoclonal antibody, 6E10, raised to the first 17 residues of the human Aβ peptide (Kim, et al. (1990) *Neuroscience Research Communicating* 7:113-122). The fusion gene product was detected in the brain, spinal cord, skeletal muscle, heart, and, minimally, lung. It was not detected in the liver, spleen, kidney, or testis.

Expression of the PrP-APP fusion gene in brain tissue was quantified by immunodot blot analysis. Relative APP expression in brain tissue was compared in transgenic and non-transgenic mice in an exponentially diluted series and reacted with antibody recognizing the 15 residues at the carboxyl terminus of APP, CT15, which recognizes both mouse and human APP (Sisodia, et al. (1993) *J*. *Neurosciences* 13:3136-3142). The total APP protein in lines of mice which developed the neurologic disease was at least 300 % of endogenous levels. Where expression was less than 300%, animals did not develop neurologic disease.

To obtain an index of brain function in affected transgenic mice, glucose utilization was regionally determined using a modification of the Sokoloff method described by Chmielowska et al. (1986) *Exp. Brain Res.* 63: 607, which allows glucose uptake/metabolism in the mouse to be measured. Regional 2-deoxyglucose concentrations determined densitometrically were normalized to the cerebellum, a region devoid of pathology. Results in transgenic mice revealed significant reductions in glucose utilization of 20-30% in the hippocampus, amygdala, and some regions of the cerebral cortex as compared to age-matched non-transgenic littermates.

### Example 4

### Analysis of Synthesis and Processing In Vitro

The synthesis and processing of the VVM717/721/722IAV mutant in cultured cells was examined to determine the effects of these mutations on disease development. The wild-type HuAPP695myc and mutant cDNA genes were cloned into the expression vector pEF-BOS (Osaka Bioscience Institute, Osaka, Japan), then transiently transfected into mouse neuroblastoma cells, which were then continuously labeled with [³⁵S]methionine for 4 hours. Labeled APP molecules were immunoprecipitated with the monoclonal antibody 22C11 (Weidemann, et al. (1989) *Cells* 57:115-126). In extracts of cells, labeled APP molecules of the appropriated size were detected in similar levels. Media from these cultures was examined for the presence of soluble APP fragments using mAb 6E10 and mAb 4G8 (Kim, et al. (1990) *supra*.). Both of these antibodies recognize the Aβ region of human APP. The mAb 6E10 recognizes sequences in Aσ between Aβ 1-17, while mAb 4G8 recognizes sequences between Aβ1-28. The sequence of Aβ17-28 is identical to mouse Aβ and thus 4G8 cannot distinguish human and mouse APP. The media of cultures transfected with either gene contained a large ectodomain fragment of APP which is routinely observed.

One of the more recent discoveries relevant to the processing of APP has been the detection of soluble Aβ 1-40 fragments in the medium of cultured cells that express HuAPP. These Aβ fragments resemble peptides found in AD amyloid plaque lesions. Thus, it appears that APP is normally processed into amyloidogenic fragments. Furthermore, mutations linked to AD have been shown to alter the processing of APP to favor the production of soluble Aβ. To determine whether the VVM717/721/722IAV mutations affected the processing of APP, the culture medium was examined for small Aβ-containing APP peptides. An Aσ peptide fragment that was immunopurified by mAb 6E10 was prevalent in the media of cells transfected with the mutant sequence. Similarly, the mAb 4G8 detected increased levels of Aβ peptide in the medium of cultures containing the mutant.

An examination of cell extracts for accumulated APP fragments detected increased levels of a 10 kDa APP peptide fragment after immunoprecipitation with anti-myc polyclonal antiserum in cells expressing the mutant (Fig. 5C, line 3). Mutations generated in mutant HuAPP695myc affect the processing of the resultant APP product to generate increased levels of soluble Aβ, and an intracellular C-terminal fragment of APP that is of sufficient length to include the Aβ region. Thus, the phenotype of animals created with the mutant APP is much like that reported for humans expressing a mutant human APP gene that encodes mutations found in a Swedish kindred of AD. To date no investigators have reported increased production of Aβ as a result of expression of HuAPP that encodes only the V642I AD-linked mutation (Golde et al., (1993), *Neuroscience* *Abstract* 19:431, 182.7). However, this mutation appears to cause a change in the length of the soluble Aβ derivative, increasing it to Aβ1-42. Thus is appears that the VVM717/721/722IAV mutations are the primary cause of the increased production of soluble Aβ. Studies on Aβ fibrillogenesis suggest that longer Aβ peptides are more amyloidogenic.

### Example 5

### Comparison of the Processing of Human and Mouse APP in Mouse Cells

Chimeric APP transgenes composed of mouse APP695 and human Aβ sequences were prepared and their processing evaluated. It is an hypothesis of the invention that there are differences in the way mouse and human APP are processed in mice. To construct humanized MoAPP cDNA, a MoAPP gene was cloned and mutated to make it compatible with the cosSHaPrP.535 vector. Mouse cDNA was isolated by reverse transcriptase-polymerase chain reaction (RTPCR), and PCR primers included *XhoI* sites at the 5' and 3' ends for cloning purposes. To remove an internal *XhoI* site in the mouse cDNA, an additional primer was included that spanned the internal *XhoI* site (codon 397) and contained a single base substitution that eliminated the *XhoI* site but preserved the correct amino acid sequence. The PCR product was subsequently sequenced to verify that unwanted mutations were not created in the PCR.

The Aβ region in HuAPP and MoAPP differs by three amino acid residues, which could affect the amyloidogenic potential of the transgene product. To humanize the mouse Aβ region, a segment of the HuAPP gene that encompassed the Aβ region was amplified by PCR using primers that include a sense primer that encompassed the *Bg*lII site at codon 590 of HuAPP-695 and an antisense primer that contained two point mutations creating a *Nar*I site at codon 626 (a cognate *Nar*I site is found in the MoAPP cDNA), while maintaining the amino acid sequence (Table 4, primers 1 and 2). This PCR product was digested with *Bgl*II and *Nar*I and then cloned into the *Bgl*II and *Nar*I sites of the MoAPP cDNA.

The chimeric (Mo/HuAPP) cDNA was sequenced across the *Bgl*II and *Nar*I sites to verify that this region now contained human Aβ sequences and to verify that no other unwanted mutations were generated. To verify that this recombinant cDNA could be expressed into full-length protein, DNA was cloned into a modified pEFBOS vector. The pEF-BOS vector contains the promoter element, first exon, first intron, and part of the second exon of the mammalian elongation factor 2α along with an SV40 origin of replication, permitting the replication of vectors and the high expression of genes in COS-1 cells. COS-1 cells were transfected with pEF-BOSMo/HuAPP-695 and cell extracts were analyzed by immunoblotting. CT15 recognized a full-length Mo/HuAPP polypeptide, whereas immunostaining with monoclonal antibody 6E10 verified that the humanized mouse cDNA product did indeed encode human Aβ sequences.

To generate chimeric Mo/HuAPP cDNA that encodes a double mutation linked to an early-onset AD, a PCR-based approach similar to that outlined above using primers 2 and 3 (Table 4) was employed. The template for the reactions was a cloned copy of Mo/HuAPP-695. The mutated chimeric gene was sequenced across the *Bgl*II and *Nar*I sites to verify the presence of mutations and to be certain that no unwanted mutations existed in the transgene. The mutant Mo/HuAPP cDNA was cloned into pEFBOS and transfected into COS-1 cells to determine whether APP polypeptides were synthesized. An APP polypeptide of the predicted size reacted with both CT15 and 6E10 antibodies.

An examination of the synthesis and processing of Mo-, Hu-, and Mo/HuAPP in mouse N2a cells has surprisingly revealed discernible differences. What is evident is that a greater percentage of MoAPP is cleaved to generate a soluble ectodomain fragment than is HuAPP. The ratio of cell-associated versus soluble moAPP is approximately 1 to 5, while 3 times more of the HuAPP is cell-associated than is soluble. The percentage of Mo/HuAPP695 that is cleaved to generate soluble ectofragments appears to fall between that of Mo- and HuAPP as the ratio of cell-associated to soluble Mo/HuAPP approaches 1 to 1. The majority of soluble APP ectofragments appear to arise from a cleavage event within Ad at the cell surface; the differences in the ratio of cell-associated APP versus soluble ecto-fragments indicate differences in the maturation of the polypeptides. Specifically, the majority of MoAPP reaches the cell surface and is cleaved by a secretase. In contrast, HuAPP may not reach the cell surface as efficiently, thus precluding secretase cleavage. The Mo/HuAPP polypeptide appears to be intermediate between Mo and HuAPP. Alternatively, it is possible that sequences within the Aβ domain influence the efficiency of secretase cleavage.

In addition to differences in the production of soluble APP ecto-fragments, differences in the level of soluble Aβ peptides were noted. All three proteins gave rise to soluble Aβ peptides that were of a size and character consistent with identification as Aβ1-40. In cells transfected with MoAPP, a fragment that is of a size and character consistent with identification as Aβ17-40 was detected. The Aβ17-40 fragment is thought to arise after membranal cleavage of APP by the putative asecretase, which cleaves between Aβ16 and -17. Only the Hu- and MoHuAPP derived Aβ1-40 peptides were recognized by mAb6E10 as expected. While MoAPP appeared to give rise to relatively equal amounts of Aβ1-40 and Aβ17-40, HuAPP and Mo/HuAPP were preferentially cleaved to generate only Aβ1-40. These results suggest that sequences differences within the human Aβ domain influence APP proteolytic cleavage.

### Example 6

### Comparison of Normal Aged Mice and Transgenic Mice Transgene Construction.

The PrP-APP transgenes were generated as described in Example 1 by replacing a *Sal*I-flanked tetracycline resistance sequence in a hamster PrP cosmid vector (Scott *et al*, (1992), *supra*), with *Sal*I-flanked human and mouse APP coding sequences. Tg mice were prepared using one of six different PrP/APP chimeric transgenes: murine wild-type APP695 (MoAPP695.WT); human APP-695 containing two mutations at M670N and K671L (APP770 numbering) (HuAPP695.SWE); human APP695 containing two mutation at M670N and K671L (APP770 numbering) (HuAPP695.SWE); human APP695 containing a mutation at E693Q (HuAPP695.DUT); human APP770 with M670N and K671L (HuAPP770.SWE); human APP695 with a triple mutation at V717I, V7211A, and M722V with a 3'-myc tag (HuApp695.TRImyc); and human wild-type APP695 with a 3'-myc tag (HuApp695.WTmyc). The CS1HuAPP695.SWE, CS1HuAPP770.SWE, CS1HUAPP695.TRImyc and CS2HuApp695.TRImyc APP sequences were modified for strong translation initiation.

Like the Swedish mutation, triple V7171I, V721A and M722V mutations in the transmembrane domain of APP enhance secretaion of Aβ by five-fold in cultured cells. The 3'-myc tag, a 12 codon segment of the c-myc proto-oncogene, was shown in cultured cells to facilitate immunodetection of transfection products (Wong and Cleveland, (1990) *The Journal of Cell Biology* 111, 1987-2003). In Tg(HuAPP695.WTmyc) and Tg(HuAPP695.TRImyc) mice the myc-tag was not as clearly detectable in Western blots and histologic samples as HuAPP reacted with human-specific APP antibodies. The myc-tag exerted no apparent effect on the phenotype, since Tg(HuAPP695.SWE),T g(HuAPP770.SWE), and Tg(HuAPP695.DUT) mice lacking the myc-tag develop the same clinical and pathologic abnormalities. The constructions were made by substituting the *Sal*I to *Kpn*I DNA sequence at the 5' end of the APP coding sequence for DNA sequences made using the polymerase chain reaction(PCR) and two sets of primers. For the CS1 APP sequence, the primer set used was 5'-AAGTCGACACCATGCTGCCC-GGTTTGGCACT-3' and 5'-AAGGTACCTCCCAGCGCCCGAGCC-3'. For the CS2 APP sequence, the primer set used was 5'-AAAAAAGTCGACACCATGGT-GCCCGGTTTGGCACT-3' and 5'-AAGGTACCTCCAGCGCCCGAGCC-3'. The HuAPP mutations were made using standard. methods of site-directed mutagenesis. Each PCR fragment synthesized for the constructions was sequenced. The PCR fragments selected for use in the constructions were free of unintended mutations. The PrP-APP cosmids were digested with *Not*I (which releases the PrP-APP fusion gene from the pcos6EMBL vector). The PrP-APP fusion genes were isolated after size fractionation on an agarose gel and electroeluted. The PrP-APP fusion gene was further purified with organic solvents, and precipitated in ammonium acetate and ethanol. The PrP-APP fusion genes were dissolved in 10mM Tris-Cl (pH 8.0) to a final concentration of 3-4 µg/ml prior to embryo injection. 1503: 5'-CTGACCACTCGACCAGGTTCTGGGT-3' and 1502: 5'-GTGGATAA-CCCCTCCCCCAGCCTAGACCA-3', located in the 3' region of APP and the 3'-untranslated region of PrP, respectively. The 1503 primer recognizes a region which is homologous in mouse and human APP, and can therefore be used to detect both PrP-MoAPP and PrP-HuAPP DNA. Using primers 1502 and 1501: 5'-AAGCGGCCAAAGCCTGGAGGGTGGAACA-3', a parallel PCT reaction amplifying a fragment of murine PrP was performed as a positive control.

Transgene copy number analysis was performed using 5µg denatured purified tail DNA baked onto nitrocellulose and hybridized to a radiolabelled 1.3kb *Sal*I-*Xho*I DNA fragment encoding a segment of the hamster PrP 3'-untranslated region located in the DNA sequence at the 5' end of the APP coding sequence for DNA sequences made using the polymerase chain reaction (PCR) and the two sets of primers described in Example 1. The HuAPP mutation were made using standard methods of site-directed mutagenesis. Each PCR fragment synthesized for the constructions was sequenced. The PCR fragments selected for use in the construction were free of unintended mutations. The PrP-APP cosmids were digested with *Not*I and the PrP-APP fusion genes were isolated after size fractionation on an agarose gel and electroeluted and further purifies as described in Example 1. The PrP-APP fusion genes were dissolved in 10mM Tris-Cl (pH8.0) to a final concentration of 3-4 µg/ml prior to embryo injection.

### Transgenic Mouse generation and screening

Transgenic lines were initiated by microinjection of single-cell mouse embryos as described (Hogan et al., (1986) *supra*). Embryo donors and fertile studs were inbred FVB mice obtained from the National Cancer Institute (NIH). Post-weaning tail biopsy DNA was generated as described (Hanley and Merlie, (1991) *Biotechniques* 10, 56). One microliter of the unpurified DNA was used in a 25µl PCR reaction. To detect PrP-APP fusion DNA, the PrP-APP fusion DNA was amplified using the polymerase chain reaction with a pair of oligomer primers, 1503: 5'-CTGACCACTCGACCAGGTTCTGGGT-3' and 1502: 5'-GTG-GATAACCCCTCCCCCAGCCTAGACCA-3', located in the 3' region of APP and the 3'-untranslated region of PrP, respectively. The 1503 primer recognizes a region which is homologous in mouse and human APP, and could therefore be used to detect both PrP-MoAPP and PrP-HuAPP DNA. Using primers 1502 and 1501: 5'-AAGCGGCCAAAGCCTGGAGGGTGGAACA-3', a parallel PCR reaction amplifying a fragment of murine PrP was performed as a positive control.

Transgene copy number analysis was performed using 5 µg denatured purified tail DNA baked onto nitrocellulose and hybridized to a radiolabelled 1.3kb *Sal*I-*Xho*I DNA fragment encoding a segment of the hamster PrP 3'-untranslated region located in the hamster PrP cosmid vector (Scott, et al., (1992) supra). After two high-stringency washes and exposure to radiosensitive film, the relative intensities of signals from genomic DNAs of transgenic mice and hamsters were compared using a phosphorimager to obtain transgene copy numbers relative to diploid hamster genomic DNA.

### Analysis of transgene expression

APP transgene product expression was examined in progeny of transgenic founders sacrificed at one to four months of age. Quantitative immunoblotting of extracts from brain homogenates was carried out in parallel with extract prepared from age-matched nontransgenic littermates. 20% (w/v) homogenates of brain tissues were prepared in TNE (50mM Tris-Cl Ph 8.0, 150 mM Na Cl, 5 mM EDTA with 2% PMSF) buffer, using a hand-held Polytron. Homogenates were diluted with an equal volume of TNE 1% N40, 1% Deoxycholate, 0.4% SDS and sonicated in a bath sonicator until all viscosity was lost. Homogenates were then boiled for 10 minutes and centrifuged at 10,000 x g for 10 minutes.

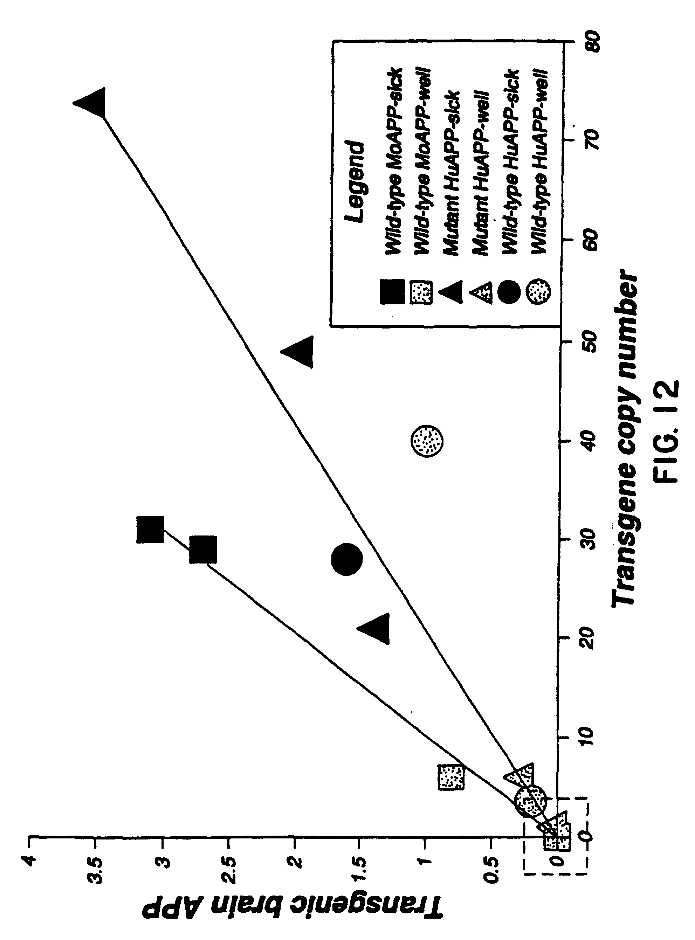

The supernatants were mixed with an equal volume of 2 X sample buffer (Laemmli, (1970) *Nature* 227, 680-685), boiled 2 min, and fractionated using a 6% SDS-PAGE. Proteins were electrophoretically transferred to Immobilon membranes (Pierce) and incubated with polyclonal (CT15 and antiGID) and monoclonal (22C11 and 6E10) APP antibodies. Reactive rabbit polyclonal antibodies were visualized following incubation with secondary rabbit antibodies to mouse IgG before incubation with ¹²⁵I-protein A. Radiointensities were quantified on a phosphorimager (Molecular Dynamics, Inc.). APP expression in brain tissue was measured in Tg mice harboring different transgene copy numbers by quantification of immunoblots in Tg lines with three antibodies recognizing both MoAPP and HuAPP, CT15 (Figure 11), anti-GID (Figure 11), and 22C11 (Figure 11). CT15 (Sisodia et al., (1993) *J. Neurosciences* 13:3136-3142; Borchelt et al., (1994) *J.Biol.Chem* 269: 14711-14714); anti-GID (Cole et al., (1989) *Brain Res. Reviews* 13:325-349); and 22C11 (Weidemann et al., (1989) *Cell* 57:115-126) recognize both mouse and human APP equally, but 22C11 also binds APLP2, a close relative of APP, with the same avidity (Slunt et al., (1994) *J.Biol. Chem* 269:2637-2644). Minor variations in HuAPP levels relative to MoAPP expression obtained with different antibodies may reflect differences in the avidity of antibody binding or distinctions in post-translational processing between wild-type and variant HuAPP. Transgenic brain APP protein expression was dependent upon copy number as well as the species of APP expressed (Figure 12). Relative to HuAPP, equivalent levels of MoAPP were achieved with lower numbers of transgene copies.

To measure the level of HuAPP specifically, brain homogenates were probed with 6E10 antibody raised against residues 1-17 of human Aβ (Kim et al., (1990) *Neuroscience Res. Comm.* 7:113-122). No reactivity to ∼ 100-125 kD APP molecules was detected in non-Tg mice (Figure 11). In Tg1130H mice the highest levels of HuAPP detected on immunoblots using 6E10 antibody were in the brain and spinal cord, and much smaller amounts (<5% of brain levels) were found in the striated muscle, heart, skin, and lung. HuAPP was poorly detected or absent in the thymus, liver, spleen, kidney, testis, and small intestine.

Specific immunostaining for human APP/Aβ using the 6E10 or 8E5 antibody *(Athena Neurosciences)* revealed HuAPP throughout the brain. 8E5 recognizes a segment of APP spanning residues 444-592 (APP695 numbering). Two different methods were used to enhance APP immunoreactivity in brain tissue from Tg lines overexpressing HuAPP. In high copy number lines, following either formic acid pretreatment of tissue using 1:5000 dilution of 6E10 antibody or microwave pretreatment of tissue using either 1:100 6E10 antibody or 1:100 8E5 antibody, APP staining was invariably present within vesicular structures in large pyramidal cells of the hippocampus, parahippocampal area, amygdala, and the cerebral cortex (Figure 13A,C,H). In some brains, fainter immunoreactivity was also visible in smaller neurons in the cortico-limbic regions of the brain and in large and small neurons of the basal ganglia, brainstem, and cerebellum. Staining was absent in non-Tg mice (Figure 13B,H) and in untreated brain tissue from affected Tg mice. The pattern of HuAPP immunostaining obtained reflected the widespread expression of HuAPP in the brain with the highest levels of expression in the telencephalon, as independently confirmed in regional brain immunoblots using the 6E10 antibody (Figure 11).

The 8E5 antibody stained amyloid plaques and intraneuronal vesicular structures in microwaved tissue sections from patients with AD (Figure 13F). At 1:5000 dilution, the 6E10 antibody stained amyloid plaques from patients with AD only after formic acid pretreatment of brain tissue (Figure 13D,E). However, in TgHuAPP mice neither the microwave nor formic acid pretreatment of brain tissue revealed HuAPP staining resembling extracellular amyloid or pre-amyloid deposits using either antibody. The abnormal phenotype in these Tg mice, therefore, was not caused by amyloid or pre-amyloid deposition.

To assess the relative effects of mutant and wild-type APP transgene expression on the development of a CNS disorder, the percentage of animals sick or dead at 100 and 200 days in lines expressing different levels of wild-type HuAPP, mutant HuAPP, or wild-type MoAPP (Table 5) was determined. These data demonstrate a direct relationship between APP expression and the development of an abnormal phenotype (Figure 15). A comparison of Tg mice expressing wild-type HuAPP and mutant HuAPP was not possible over the full range of APP expression. However, a comparison of TG mice expression approximately two to four fold mutant HuAPP, (TgHuAPP695.TRImyc)1140 and (TgHuAPP695.TRImyc)1130, with Tg mice expressing approximately three fold wild-type MoAPP, (TgMoAPP695.WT)1874, indicates that mutant HuAPP will readily provokes the abnormal phenotype. This observation argues against the abnormal phenotype being due to a non-specific effect of Tg protein over expression, since mutant HuAPP conferred the disorder with higher penetrance than wild-type MoAPP, demonstrating a specific effect of the Tg protein species it expressed. These data are represented as titration curves that demonstrate a direct relationship between APP expression and the development of an abnormal phenotype (see Figure 15). However, the left-shifted curve for Tg mice expressing mutant APP relative to wild-type APP indicates that expression of the mutant APP more readily provokes the abnormal phenotype.

To ensure that overexpression of a foreign (human) species of protein did not artefactually produce the abnormal phenotype, Tg mice overexpressing wild-type MoAPP were generated. In Tg mice with MoAPP levels equivalent to 3.1-fold endogenous APP levels the same phenotype occurred, indicating that the observed phenotype was not due to overexpression of a foreign species of protein.

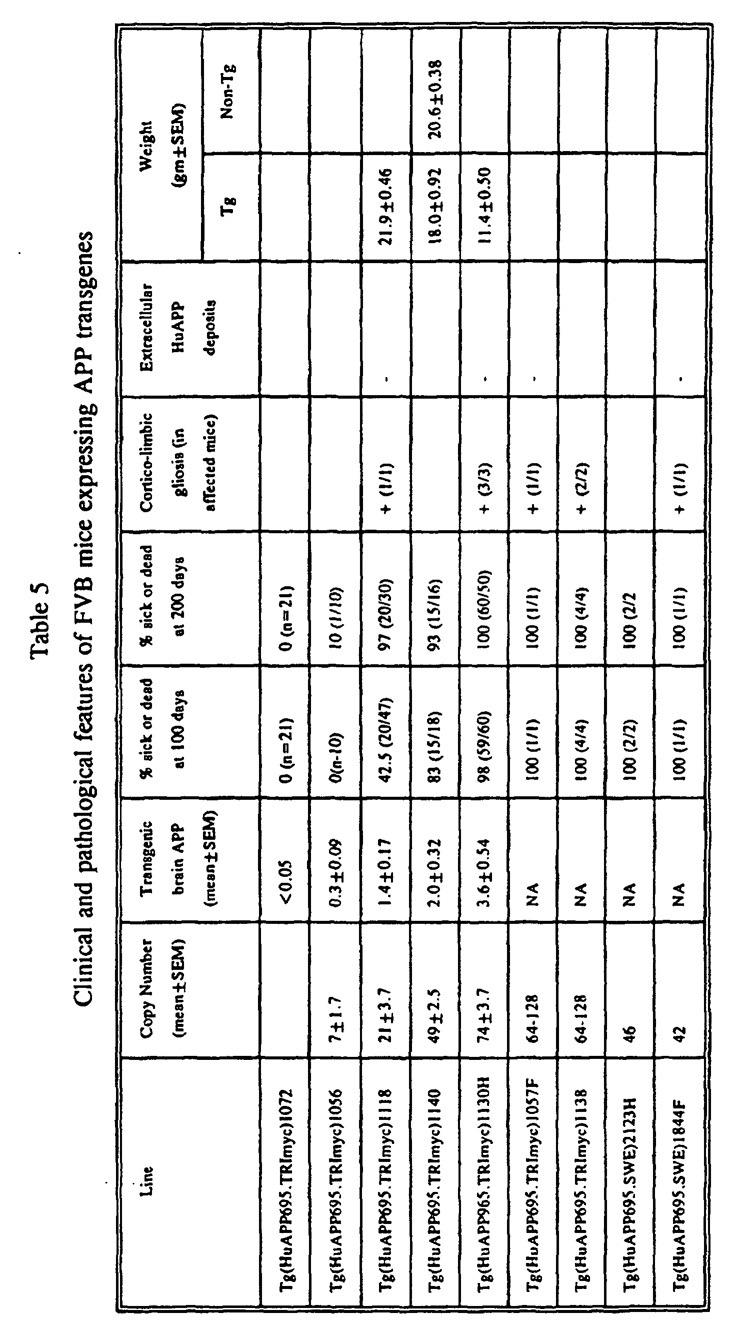

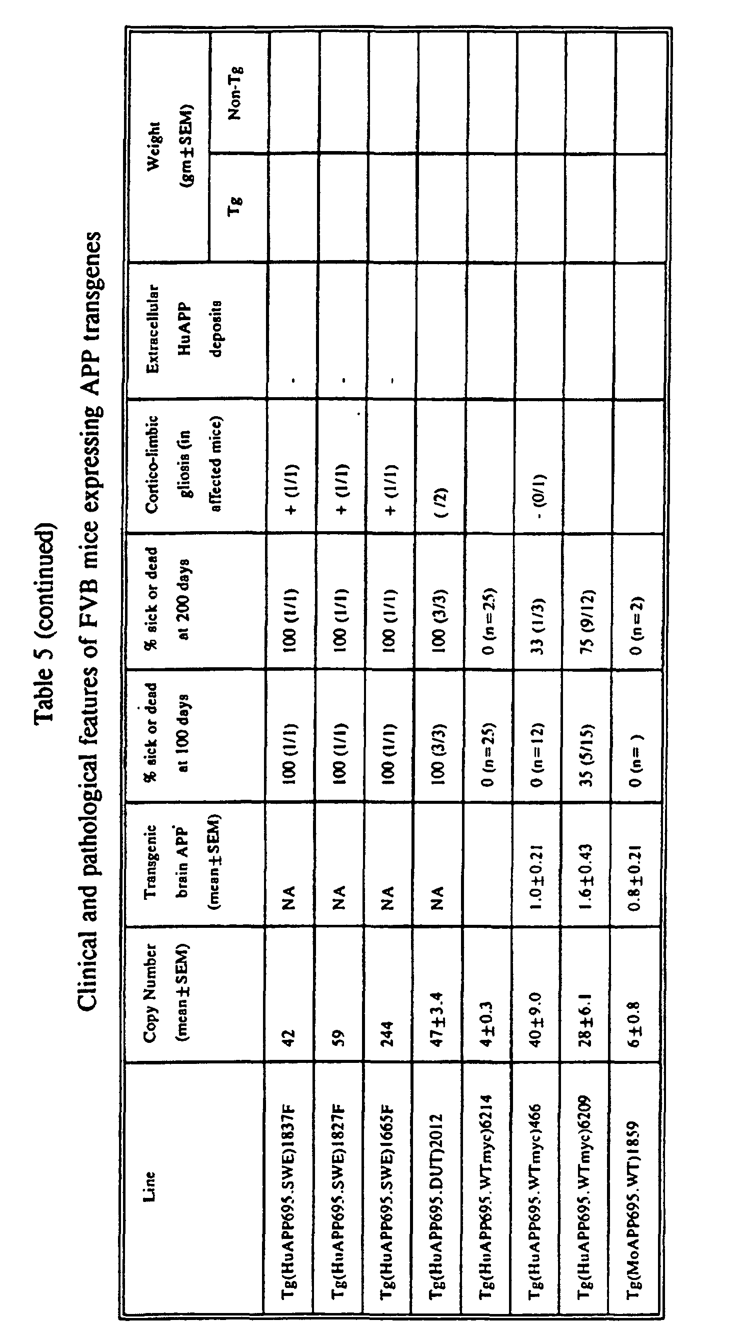

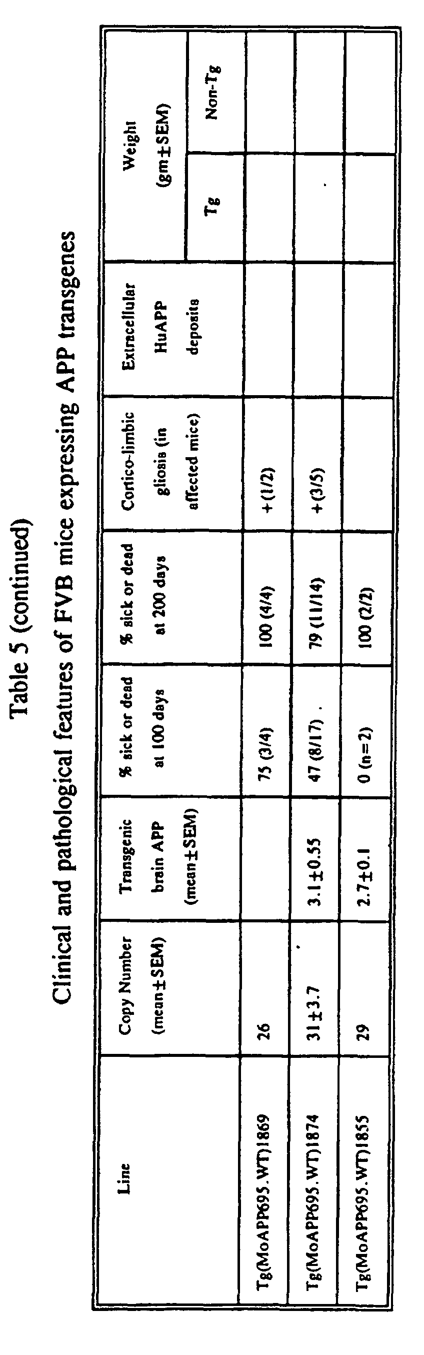

### Behavioral analyses

To determine whether FVB mice naturally became behaviorally impaired with advancing age (the mouse equivalent of senile dementia in humans), FVB mice were observed up to one year and the behavior of these aged mice compared to that of transgenic mice. Behavioral analyses were usually performed three times per week using the comer index (CI) test. The test exploits a striking neophobic response which occurs in many affected Tg mice. The neophobic response is manifested by a decrease in exploratory activity specific to testing in a novel chamber. Early in the clinical course, affected mice often appear normal in their home cages but exhibit transient immobility for 30 to 60 seconds after being placed alone in a clean cage, in contrast to unaffected mice which typically explore and sniff around the novel setting. A characteristic response of an affected mouse is to hold its neck low with its tail stiff during the transient immobility. Alternatively, an affected mouse runs to a comer and then assumes a crouched or frozen posture there. The (CI) test measures the number of times a mouse sniffs the comers of a clean cage during the first 30 seconds after it is placed alone into that cage. Based upon the collective observations of > 2000 tests of > 100 Tg mice and > 2500 tests of > 140 non-Tg mice, we established criteria for the presence of a behavioral disorder were determined to be scores of two "0's" or "0 and 1" occurring within three consecutive tests. The onset of illness is ascribed to the first of three consecutive testing dates in which abnormal scores are obtained.

To perform the comer index test, a test mouse, held by the tail, is placed in the center of a clean cage that is otherwise identical to its home cage. The number of times the mouse sniffs the corners of the test cage during the first 30 seconds after it was placed into that cage are recorded as the CI. Animals which are obviously moribund before attaining the CI criteria and animals which develop witnessed seizures also are diagnosed as ill. Animals housed alone are excluded from the analysis because several non-Tg and Tg mice obtain low scores while housed alone without displaying the characteristic freezing postures of the affected Tg animals. When these mice are housed with other mice, their CI scores increase. To control the variations in diurnal activity, all animals are tested between 1430h and 1830h.

### An age-related CNS disorder in FVB mice Behavioral abnormalities.

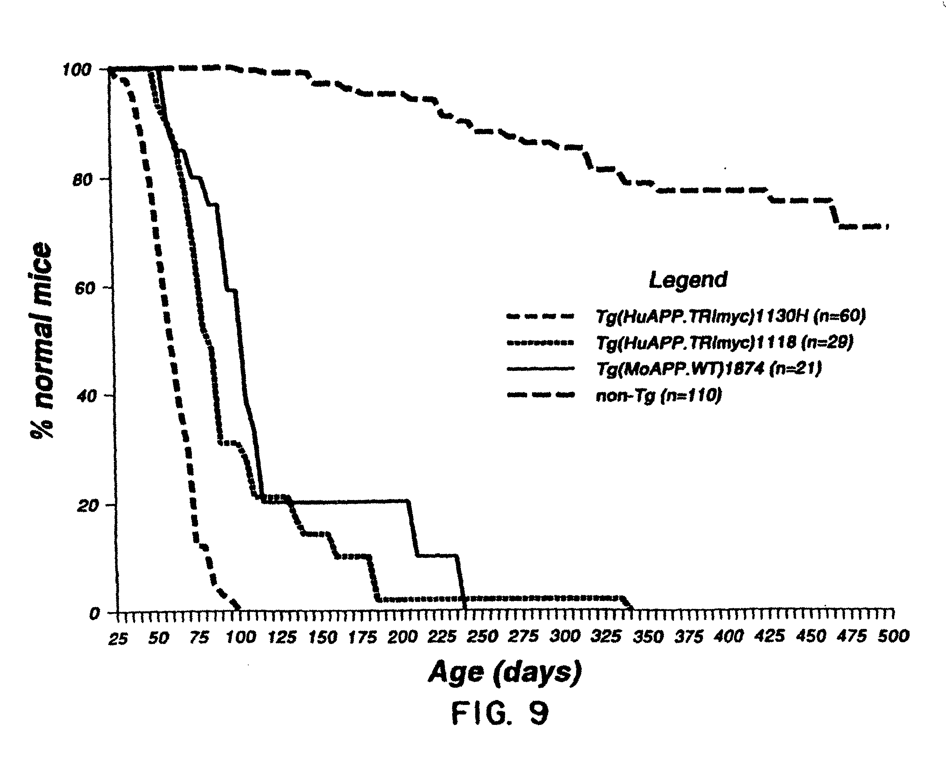

The life expectancy of FVB mice is approximately 600 days but little is known about age-related CNS disorders in FVB mice. To determine whether FVB mice naturally become behaviorally impaired with advancing age, 110 FVB mice 150-500 days of age from three different institutions (University of Minnesota, Minneapolis, MN, McLaughlin Research Institute, Great Falls, MT, and Harlan Sprague Dawley, Inc. Indianapolis, IN) were observed. With advancing age, 18 mice as early as 154 days of age developed behavioral abnormalities, including agitation, inactivity, seizures, and neophobia, as defined by the corner index test, and premature death (Table 6). Another six mice died from tumors or accidentally. Although agitation or inactivity occurred in all affected Tg mice, these were subjective signs that rarely appeared in most normal mice. the onset of illness was defined by corner index test results in conjunction with the observation of seizures, agitation or apathy. Both male and female mice were affected. Three agitated mice died prior to diagnosis by corner index criteria. One death occurred immediately following an observed seizure. The remaining mice grew progressively less active, and were sacrificed for pathologic studies between nine and 91 days after the onset of abnormal behavioral signs. The cumulative incidence of behavioral abnormalities and death (excluding accidental and tumor-related deaths) in this cohort of FVB mice was 23% by 500 days of age (See Figure 9).

*Gliosis.* Brains from sixteen older non-Tg FVB mice nine to twelve months of age, seven exhibiting the abnormal behavior characteristic of affected Tg APP mice and nine age-matched behaviorally normal mice, were examined in a coded fashion. Six of the seven brains from the behaviorally abnormal mice exhibited profound hypertrophic astrocytic gliosis in the hippocampus, prprhippocampal area, amygdala, and cerebral cortex (Figure 10). None of the brains from the nine age-matched, behaviorally normal mice exhibited this degree of gliosis, although moderate gliosis restricted to the hippocampus was observed in some mice. These findings indicate that the behavioral disorder in affected older non-Tg mice is tightly associated with cortico-limbic gliosis (Yates-corrected X²=8.96, p=0.003). The brains of the non-Tg behaviorally impaired FVB mice showed no amyloid plaque deposition, neurofibrillary tangle formation, neuronal abnormalities, or qualitative changes in neuronal or glial numbers.

*Regional-cerebral glucose utilization*. To obtain an independent functional assessment of the abnormal behavior observed in impaired FVB mice, regional brain glucose utilization was determined using a modification of the Sokoloff method (Sokoloff et al., (1977) *J*. *Neurochem* 28, 897-916). Regions associated with learning, memory, and emotion such as the cerebral cortex, hippocampus, entorhinal cortex, and amygdala, which are most impaired in cognitively impaired aged humans and patients with AD were examined. Densitometric values of ¹⁴C-deoxyglucose distribution were normalized to cerebellar values because the cerebellum appeared uninvolved clinically and pathologically. The regional cerebral glucose utilization in cerebral tissue in impaired FVB mice was compared to that in cerebral tissue in behaviorally normal, age-matched FVB mice. Significant decreases (p < 0.05, analysis of variance) in regional glucose utilization, particularly in the hippocampus (-42%), amygdala (-43%), entorhinal cortex (-46%), parietal cortex (-34%), frontal cortex (-19%) and temporal cortex (-18%), were observed in the cerebral tissue in the impaired FVB mice. In contrast, no significant decreases were observed in several structures, including the corpus callosum, medullary reticular formation, dentate nucleus, and vermis.

The development of impaired behavior accompanied by cortico-limbic hypertrophic gliosis and diminished regional cerebral glucose utilization, especially in the cerebrum, in FVB mice defines a characteristic age-related CNS disorder with features of the senescent changes observed in other rodent species, such as hypertrophic gliosis and diminished regional glucose utilization in limbic and cortical structures. Although the age-related behavioral abnormalities observed in impaired FVB mice have not been described to occur naturally in other rodents, the major decrease in regional cerebral glucose utilization found in the corticolimbic areas of the brain involved in learning, memory, and emotion, strongly suggest that some, if not most, of the behavioral abnormalities in affected FVB mice reflect dysfunction in these brain regions. Because the behavioral, pathological, and functional abnormalities observed in these mice share features found in other aged, impaired rodents and in demented humans, the constellation of findings represents a form of CNS senescence in FVB mice.

### Transgenic mice expressing mutant and wild-type APP

*Behavioral abnormalities*. An abnormal phenotype resembling that in aged, impaired FVB mice developed in animals expressing high levels of APP. Copy number *per se* was unlikely to be the direct cause of the CNS disorder, since a previously published Tg line developed in FVB mice, Tg(HuPrP)FVB-152, expressing human PrP driven by 30-50 copies of the hamster PrP gene cosmid exhibited no premature behavioral abnormalities or death (Telling et al., (1994) *Proc*.*Natl*.*Acad*.*Sci*. *U.S.A.* 91,9936-9940). The phenotype in TgAPP mice segregated according to the species, genotype and level of APP expression in four lines harboring roughly equivalent copy numbers (20-30): Tg(HuAPP695.WYmyc)466, Tg(MoAPP695.WTmyc)6209. to determine whether PrP levels were affected by the presence of supernumerary PrP gene components, brain PrP levels were measured in Tg(HuAPP695.TRImyc) 1130 mice with 74 transgene copies and non-Tg mice. No differences were found, indicating that alterations in PrP expression were also not the cause of the abnormal phenotype.

Affected Tg animals developed all the clinical signs observed in aged, impaired non-Tg FVB mice, including agitation, increased startle responses, apathy, and neophobia (Table 6), but they occurred with significantly high penetrance at earlier ages (Figure 9, Table 5). Later in the course inactivity and failure to reproduce developed but there was no tremor, incoordination, weakness, paralysis, or apparent loss of sensation as judged from their withdrawal or vocal responses to tail or foot pinching. Seizures were observed in a small percentage (3% (6/181)) of affected Tg(HuAPP695.TRImyc) mice. It is possible that the actual incidence of seizures is higher, and would be detected if mice were observed for more than 30-60 seconds three times per week.

Behavioral abnormalities in Tg mice developed as early as one month of age. There was no significant difference between the onset of behavioral abnormalities in male and female mice. Some Tg mice (=14%) overexpressing APP died as early as one month of age without exhibiting prior seizures or neophobia. A neuropathologic examination of two of these mice identified corticolimbic gliosis indistinguishable from Tg mice that had died after exhibiting the characteristic behavioral signs, so it is probable that these mice died as a result of the same disorder as the other affected Tg mice.

Small stature was observed in animals with transgenic brain APP levels exceeding twice the endogenous levels (Table 5). This difference in size was not apparent at birth became conspicuous by four to six weeks of age, and was less or absent in older animals. The Tg animals appeared normally proportioned. Small size was no required for behavioral abnormalities to occur, since Tg(HuAPP695.TRImyc)1118 mice died prematurely and developed behavioral abnormalities despite being normal in size.

**Table 6.**

| Clinical and pathological signs in aged, impaired FVB mice and in affected FVB mice expressing APP transgenes | | |
|---|---|---|
| **Signs** | **% aged, impaired FVB mice** | **% affected Tg FVB mice** |
| Seizures | 17% (3/18) | 3% (6/181) |
| Agitation or inactivity | 100% (18/18) | 100% (181/181) |
| Neophobia | 83% (15/18) | 84% (152/181) |
| Early death (excluding sacrificed mice) | 100% (4/4) | 100% (82/82) |
| Cortico-limbic gliosis | 86% (6/7) | 76% (16/21) |

### Pathological analyses of transgenic mice

Brains of transgenic mice exhibiting behavioral abnormalities or found dead and age-matched nontransgenic littermates were examined for neuropathologic abnormalities. Brains were immersion fixed or perfused with 10% phosphate-buffered formalin or 4% buffered paraformaldehyde, embedded in paraffin, and cut into 5-8 µm sections on a rotary microtome. Tissue sections were stained with hematoxylin and eosin, cresyl violet, thioflavin S, or Congo Red stains, or using the Bielschowsky silver or TUNEL (Gavrieli et al., (1992) *Journal of Cell Biology* 119, 493-501) methods.

For immunohistologic studies, paraffin sections were deparaffinized and rehydrated through xylol and graded alcohols. Endogenous peroxidase was quenched by treatment with 6% hydrogen peroxide in methanol for 10 minutes or with 3.0% hydrogen peroxide in methanol (1:5), and rinsed in deionized water or phosphate buffered saline. To enhance APP antigen detection, selected sections were microwave irradiated in water at full power for 15 minutes, cooled to room temperature, transferred to deionized water in 0.5 M TBS (pH 7.6), and pretreated with 0.4% TX/TBS, followed by 3% normal goat serum in TBS. Primary antibodies 6E10 (1:100) and 8E5 (1:100 ascites fluid) were prepared in 0.1% TX/TBS with 2% normal goat serum.

Following incubation for 24 hours, slides were rinsed, incubated in goat-antirabbit or -antimouse IgG (1:20) in 0.1% TX/TBS, and rinsed in TBS followed by one-hour incubation in rabbit or mouse peroxidase-antiperoxidase (1:100) at room temperature. Rinsed slides were reacted in the presence of 0.05% diaminobenzidine in 0.01% hydrogen peroxide, rinsed three times in TBS, dehydrated through a graded series of alcohols to xylene. Representative sections were silver-enhanced according to the Fontana-Masson method (Masson (1928) *Am. J*. *Path*. H: 181-211), and viewed under transmitted light microscopy and differential interference contrast optics. Other sections were immersed in 70% formic acid for 10 minutes, rinsed in phosphate buffered saline, and immersed in 10% normal horse serum for 1 hour. Primary antibody 6E10 (1:5000) was prepared in phosphate buffered saline. Following incubation overnight at 4°C, sections were rinsed in phosphate buffered saline, incubated with antimouse IgG, followed by avidin-biotin complex (Vector Labs, Inc). Rinsed slides were reacted with diaminobenzidine and counterstained with Harris hematoxylin. GFAP was detected using a monoclonal antibody to GFAP at a dilution of 1:60 in phosphate buffered saline.

*Gliosis.* Using coded specimens, brains from 21 affected Tg mice expressing the triple HuAPP variant, the Dutch HuAPP variant, the Swedish HuAPP variant, wild-type HuAPP, as well as brains from 12 age-matched, unaffected non-Tg mice were examined. Brains from 16 affected Tg mice exhibited prominent hypertrophic astocytic gliosis located predominantly in the parahippocampal area, hippocampus, amygdala, and cerebral cortex (Figure 10), with relative sparing of the basal ganglia. The astrocytes had enlarged, elongated processes when immunostained for glial fibrillary acid protein (GFAP), but there was no increase in the number of astrocytes. Brains from the age-matched non-Tg mice were devoid of the reactive gliosis, indicating a strong association between gliosis and abnormal behavior (Yates-corrected X²=14.83, p=0.00012). Bielschowsky silver stains revealed no neurofibrillary tangles, dystrophic neurites, or neuritic plaques. Neurons appeared normal with Nissl and hematoxylin and eosin stains.

Gross and microscopic examinations of six Tg mice found dead revealed characteristic brain pathology (astrocytic gliosis in the hippocampus, cerebral cortex, amygdala, and parahippocampal area, as described below), but no evidence of microscopic or gross pathology outside the CNS. Amyloid was specifically excluded by thioflavin S staining in the heart, lung, liver, spleen, thymus, kidney, small intestine, and testes in four of these Tg mice. The absence of pathologic findings outside the CNS indicates that the deaths were most likely due to causes which were neurologic in origin.

### Regional cerebral glucose utilization

To determine whether there were functional differences in the brains of affected Tg mice, regional brain glucose utilization was compared among affected Tg mice with aged, impaired non-Tg FVB mice and age-matched non-Tg mice. Compared to normal, non-Tg littermates, significant reductions (p < 0.05; analysis of variance) in glucose utilization were observed in various forebrain regions in Tg mice, including the hippocampus (-31%), amygdala (-28%), parietal cortex (-34%), temporal cortex (-33%), and occipital cortex (-36%). Many regions, in contrast, showed no significant reduction (p > 0.05), including the sensory-motor cortex, corpus callosum, reticular formation, vermis, vestibular complex, and dentate nucleus. The diminution of regional glucose utilization was particularly pronounced in the hippocampus, amygdala, and some cortical regions in affected Tg mice closely resembling that occurring in older, impaired non-Tg FVB mice.

### Extracellular Aβ staining in a Tg mouse.

One animal shows extracellular staining with an antibody described in Saido et al., *JBC* 269 (21) :15253-15257, 1994. This antibody specifically stains the aminoterminus of Aβ. It is an affinity purified polyclonal antibody. The staining in our Tg mouse can be blocked by specific competition with the Aβ fragment. The staining pattern in our Tg mouse resembles that which is seen in AD brain stained with the same antibody. More animals are being examined. Further characterization with other antibodies is being done. Ultrastructural studies also being done.

### Example 7

### Testing for Drugs That Prevent Progressive Neurologic Disease

The animals of the invention are used to test materials for the ability to confer protection against the development of progressive neurologic disease. An animal exhibiting the progressive neurologic disease is treated with a test material in parallel with an untreated control transgenic animal exhibiting the neurologic disease. A comparatively lower incidence of the progressive neurologic disease in the treated animal is detected as an indication of protection. Treated and untreated animals are analyzed for diminished exploratory/locomotor behavior (CI test; see Example 6), as well as diminished 2-deoxyglucose uptake/utilization and hypertrophic gliosis in the cortico-limbic structures of the brain. To determine if a treatment can prevent or delay the onset of disease, half of the transgenic mice in a litter from a line of mice known to develop neurologic illness may be randomly assigned to receive the treatment, and the other half to receive a placebo, beginning at an age prior to the earliest known onset of disease for the given line of mice. The number of litters to be used will depend upon the magnitude of the differences observed between treated and untreated mice.

Mice are observed daily; their diagnosis is facilitated by the use of the CI test (see Example 6) which is administered three times per week by individuals blinded to the experimental groups. Survival curves and mean ages of disease onset and death are calculated form the accumulated clinical data.

Clinical results are corroborated by performing neuropathologic and glucose-uptake studies in samples in the experimental and control groups. Gliosis is evaluated in immunohistologic studies using antibodies to glial fibrillary acidic protein. Glucose-uptake studies are performed using a modification of the Sokoloff method described by Chmielowska et al. (1986) *Exp*. *Brain Res.* 63:607.

To determine if a treatment can ameliorate or cure disease, sick littermates are randomly assigned to receive the treatment of interest or a saline placebo. Survival and clinical improvement on the CI test coupled with neuropathologic and glucose-uptake studies are ascertained, as described above.

### Example 8

### Testing for Drugs That Cure Progressive Neurologic Disease

The animals of the invention are used to test materials for the ability to improve or cure progressive neurologic disease. An animal exhibiting the progressive neurologic disease is treated with a test material in parallel with an untreated control transgenic animal exhibiting the neurologic disease. A comparatively delayed death, or an improvement in the neurobehavioral, pathologic, or functional indications of the disease is detected as an indication of protection. Treated and untreated animals are analyzed for diminished exploratory/locomotor behavior, as well as diminished 2-deoxyglucose uptake/utilization and hypertrophic gliosis in the cortico-limbic structures of the brain.

As demonstrated by the above results, the clinical and pathologic findings in non-human mammals with super endogenous levels of either mutant or native amyloid precursor protein show an unexpected, but striking parallel to these in humans with progressive neurologic disorders such as Alzheimer's disease; the involved regions of the neocortex in affected transgenic mice and humans are similar. In addition, glucose uptake in the sensorimotor area of the cerebral cortex was unaffected by the neurologic disease in transgenic mice. This was the only region of mouse neocortex sampled which represented mainly primary neocortex, rather than a mixture of primary and association neocortex. It is a well-known observation that in brains of patients with Alzheimer's disease, the primary neocortex is relatively free of neuropathologic findings compared to the association cortex.

The CNS phenotype of the Tg mice closely resembles the CNS phenotype of a subset of aged non-Tg mice of the same FVB strain. The gliosis in the hippocampus astrocytic gliosis that is characteristically found in the hippocampal formations of aged, memory-deficient rats (Landfield et al. (1977) *J. Gerontology* 32, 2-12) and aged, nude mice (Mandybur et al., (1989) *Acta Neuropathol (Berl.)* 77, 507-513). The regional glucose hypometabolism in both the affected Tg mice and the aged, impaired non-Tg mice was markedly diminished in the hippocampus, cerebral cortex, and amygdala, resembling the pattern of glucose gypometabolism occurring in humans with AD (de Leon et al. (1983) *Am J. Neuroradiology* 4, 568-571), and in restricted areas of the limbic system in aged, impaired Sprague-Dawley rats (Gage et al. (1984) *J. Neuroscience* 11, 2856-2865). The striking similarities in the neurologic disease exhibited by the Tg animals and the naturally occurring disorder in older mice of the same strain support the use of these Tg mice as a model for progressive senescent disorders of the brain, including Alzheimer's disease.

Animals dying of neurologic disease exhibited hypertrophic gliosis in the hippocampus, amygdala, and some areas of the cerebral cortex. Immunohistologic mapping of HuAPP in the transgenic mice indicated widespread expression throughout the brain. However, the behavioral abnormalities corresponded to the circumscribed regions of gliotic pathology and glucose hypo-utilization found in select forebrain regions. The striking similarities in target cell specificities in cortico-limbic areas of the brain (hippocampus, amygdala, and some areas of cerebral cortex) in these transgenic mice and Alzheimer's disease support the use of these transgenic mice as a model for progressive neurologic disorders such as Alzheimer's disease.

In summary, these transgenic mice express supra-endogenous levels of APP. In the lines which develop neurologic disease, APP transgene product expression with at least 200% of endogenous levels have been attained, or more than double that reported in any prior publications. More importantly, these mice have a definite, progressive neurologic disorder. Even where APP expression has been achieved in other transgenic mice, they have not developed a progressive disease affecting the cortico-limbic areas of the brain.

All publications and patent applications mentioned in this specification are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. A method for making a transgenic non-human mammal with progressive neurologic disease in corticolimbic areas of the brain, said method comprising chromosomally incorporating an expression cassette which comprises an amyloid precursor protein coding sequence operably joined to regulatory sequences obtainable from a prion protein gene which provide for expression of said coding sequence in neurologic tissues at a level at least two to four-fold that of endogenous levels of wild-type amyloid precursor protein into the genome of a non-human mammal.

2. The method according to claim 1, wherein said coding sequence is a human coding sequence.

3. The method according to claim 1 or 2, wherein coding sequence is a disease-linked mutated coding sequence.

4. The method according to claim 3, wherein said coding sequence is selected from the group consisting of CS1HuAPP695.V717I. V721A.M722V.; CS2HuAPP695.V717I. V721A.M722V.; and CS1HuAPP695.K670N.M671L wherein the translation initiation sequence CS1, is GTCGACACCATGC and the translation initiation sequence CS2 is GTCGACACCATGG.

5. The method according to any one of claims 1 to 3 wherein said coding sequence is a chimeric coding sequence.

6. The method according to claim 5 wherein said chimeric coding sequence is a human-mouse chimeric coding sequence.

7. The method according to any one of claims 1 to 6, wherein said regulatory sequence includes a sequence selected from ACCATG, ACCATG, ACCATGG, and ACCATGG, wherein ATG is the initiation codon.

8. A method according to any one of claims 1 to 7 wherein said expression cassette comprises a region of DNA flanking the prion regulatory sequences whereby copy number-dependent transgene expression is obtained.

9. The method according to any one of claims 1 to 8 wherein said progressive neurologic disease is a neurobehavioral disorder with gliosis and one or both of diminished glucose uptake and glucose utilization.

10. The method according to any one of claims 1 to 9 wherein at least twenty copies of the expression cassette are incorporated into the genome.

11. A zygote of a non-human mammal comprising:
an expression cassette as defined in any one of claims 1 to 8.

12. A zygote according to claim 11 wherein said zygote is derived from crossing rodents of the same species.

13. The zygote according to claim 12, wherein said rodents are mice.

14. The zygote according to claim 13, wherein said mice are FBV mice.

15. The zygote according to claim 13, wherein said mice are different strains.

16. The zygote according to claim 15, wherein said strains are a Swiss Webster and a CS7B16/DBA-ZF1 hybrid.

17. An embryo developed from the zygote according to any one of claims 11 to 16.

18. An animal developed from the embryo according to claim 17.

19. A cosmid vector comprising an expression cassette as defined in any one of claims 1 to 8.

20. A transgenic non-human mammal whose germ cells and somatic cells comprise an expression cassette which provides a level of expression of a mutant amyloid precursor protein which produces progressive neurologic disease in cortico limbic areas of the brain of said mammal, wherein said expression cassette is as defined in any one of claims 1 to 8.

21. A transgenic non-human mammal as claimed in claim 20, said mammal exhibiting an amount of mutant amyloid precursor protein expression in neurologic tissues of said mammal sufficient to produce impaired performance of said mammal in memory and learning tests and to induce abnormal neuropathology in a cortico-limbic region of said mammal's brain, wherein said impaired performance and said abnormal neuropathology are as compared to control mammals.

22. Progeny of said transgenic non-human mammal according to claim 20 or claim 21, wherein the germ cells and somatic cells of said progeny comprise said expression cassette.

23. A method for screening for an agent which ameliorates symptoms of Alzheimer's disease, said method comprising:
comparing performance in memory and learning tests of a first transgenic non-human mammal of claim 20 or claim 21 contacted with said agent and a second transgenic non-human mammal of claim 20 or claim 21 not contacted with said agent, whereby an agent which ameliorates said symptoms is identified by superior performance of said first transgenic non-human mammal as compared to said second transgenic non-human mammal in said memory and learning tests.

24. A method for screening for an agent useful for treating Alzheimer's disease, said method comprising:
comparing performance in memory and learning tests of a first transgenic non-human mammal of claim 20 or claim 21 contacted with said agent and a second transgenic non-human mammal of claim 20 or claim 21 not contacted with said agent; and
comparing neuropathology in a cortico-limbic region of the brain of said first and said second transgenic non-human mammals when said first transgenic non-human mammal exhibits superior performance in said memory and learning tests as compared to said second transgenic non-human mammal, whereby an agent which is useful for treating Alzheimer's disease is identified by a decrease in neuropathologic findings in said first transgenic non-human mammal as compared to said second transgenic non-human mammal.

25. A method for screening for an agent useful for treating Alzheimer's disease, said method comprising:
comparing performance in memory and learning tests of a first transgenic non-human mammal of claim 20 or claim 21 contacted with said agent and a second transgenic non-human mammal of claim 20 or claim 21 not contacted with said agent; and
comparing age of death of said first and said second transgenic non-human mammals when said first transgenic non-human mammal exhibits superior performance in said memory and learning tests as compared to said second transgenic non-human mammal, whereby an agent which is useful for treating Alzheimer's disease is identified by a greater age at death of said first transgenic non-human mammal as compared to said second transgenic non-human mammal.

26. A method for screening for an agent which ameliorates symptoms of Alzheimer's disease, said method comprising:
comparing exploratory behaviour or locomotor behaviour of a first transgenic non-human mammal of claim 20 or claim 21 contacted with said agent and a second transgenic non-human mammal of claim 20 or claim 21 not contacted with said agent, whereby an agent which ameliorates said symptoms is identified by less impaired exploratory or locomotor behaviour in said first transgenic non-human mammal as compared to said second transgenic non-human mammal.

## Patentansprüche

1. Methode zum Präparieren eines transgenen nicht-humanen Säugers mit einer progressiven neurologischen Erkrankung in den cortico-limbischen Regionen des Gehirns, welche Methode den chromosomalen Einbau einer Expressionskassette umfasst, die eine Codierungssequenz eines Amyloid-Vorläuferproteins enthält, welche funktionsfähig geknüpft ist an regulatorische Sequenzen, gewinnbar von einem Prion-Protein-Gen, die für die Expression der Codierungssequenz in neurologischen Geweben in einer Menge vom mindestens Zwei- bis Vierfachen der endogenen Mengen an Wildtyp-Amyloid-Vorläuferprotein in das Genom eines nicht-humanen Säugers sorgen.

2. Methode nach Anspruch 1, wobei die Codierungssequenz eine humane Codierungssequenz ist.

3. Methode nach Anspruch 1 oder 2, wobei die Codierungssequenz eine mit einer Krankheit verbundene mutierte Codierungssequenz ist.

4. Methode nach Anspruch 3, wobei die Codierungssequenz ausgewählt ist aus der Gruppe, bestehend aus CS1HuAPP695.V7171. V721A.M722V.; CS2HuAPP695.V7171. V721A.M722V.; und CS1HuAPP695.K670N.M671L, wobei die Translationsinitiationssequenz CS1 ist GTCGACACCATGC und die Translationsinitiationssequenz CS2 ist GTCGACACCATGG.

5. Methode nach einem der Ansprüche 1 bis 3, wobei die Codierungssequenz eine chimäre Codierungssequenz ist.

6. Methode nach Anspruch 5, wobei die chimäre Codierungssequenz eine Mensch-Maus-chimäre Codierungssequenz ist.

7. Methode nach einem der Ansprüche 1 bis 6, wobei die regulatorische Sequenz eine Sequenz enthält, ausgewählt aus ACCATG, ACCATG, ACCATGG und ACCATGG, worin ATG das Initiationscodon ist.

8. Methode nach einem der Ansprüche 1 bis 7, wobei die Expressionskassette eine DNA-Region umfasst, die die regulatorischen Sequenzen des Prions flankiert, wodurch eine Kopienzahl-abhängige Transgen-Expression erreicht wird.

9. Methode nach einem der Ansprüche 1 bis 8, wobei die progressive neurologische Erkrankung eine "neurobehavioral" Störung mit Gliosis und einem oder beidem von verminderter Glucoseaufnahme und Glucoseumsatz ist.

10. Methode nach einem der Ansprüche 1 bis 9, wobei mindestens zwanzig Kopien der Expressionskassette in das Genom eingebaut werden.

11. Zygote eines nicht-humanen Säugers, umfassend:
eine Expressionskassette, wie in einem der Ansprüche 1 bis 8 definiert.

12. Zygote nach Anspruch 11, wobei die Zygote vom Kreuzen von Nagern derselben Spezies hergeleitet ist.

13. Zygote nach Anspruch 12, wobei die Nager Mäuse sind.

14. Zygote nach Anspruch 13, wobei die Mäuse FBV-Mäuse sind.

15. Zygote nach Anspruch 13, wobei die Mäuse von unterschiedlichen Stämmen sind.

16. Zygote nach Anspruch 15, wobei die Stämme ein Swiss-Webster- und ein CS7B16/DBA-ZF1-Hybrid sind.

17. Embryo, entwickelt aus der Zygote nach einem der Ansprüche 11 bis 16.

18. Tier, entwickelt aus dem Embryo nach Anspruch 17.

19. Cosmid-Vektor, umfassend eine Expressionskassette, wie in einem der Ansprüche 1 bis 8 definiert.

20. Transgener nicht-humaner Säuger, dessen Keimzellen und Körperzellen eine Expressionskassette umfassen, die einen Expressionspegel eines mutanten Amyloid-Vorläuferproteins erbringt, welches die progressive neurologische Erkrankung in cortico-limbischen Regionen des Gehirns des Säugers erzeugt, wobei die Expressionskassette wie in einem der Ansprüche 1 bis 8 definiert ist.

21. Transgener nicht-humaner Säuger nach Anspruch 20, welcher Säuger eine Menge an Expression eines mutanten Amyloid-Vorläuferproteins in neurologischen Geweben des Säugers zeigt, die ausreichend ist, um eine beeinträchtigte Leistung des Säugers bei Gedächtnis- und Lerntests zu erzeugen und eine abnormale Neuropathologie in einer cortico-limbischen Region des Gehirn des Säugers zu induzieren, wobei die beeinträchtige Leistung und die abnormale Neuropathologie im Vergleich zu Kontroll-Säugern vorliegt.

22. Nachkommen des transgenen nicht-humanen Säugers nach Anspruch 20 oder Anspruch 21, wobei die Keimzellen und Körperzellen der Nachkommen die Expressionskassette enthalten.

23. Methode zum Screening auf ein Agens, welches Symptome von Alzheimer-Krankheit mildert, welche Methode umfasst:
Vergleichen der Leistung bei Gedächtnis- und Lerntests eines ersten transgenen nicht-humanen Säuqers nach Anspruch 20 oder Anspruch 21. der mit dem Agens kontaktiert wurde, und eines zweiten transgenen nicht-humanen Säugers nach Anspruch 20 oder Anspruch 21, der nicht mit dem Agens kontaktiert wurde,
wobei ein Agens, welches die Symptome mildert, durch eine überlegene Leistung des ersten transgenen nicht-humanen Säugers im Vergleich zum zweiten trangenen nicht-humanen Säuger bei den Gedächtnis- und Lerntests identifiziert wird.

24. Methode zum Screening auf ein Agens, welches zur Behandlung von Alzheimer-Krankheit nützlich ist, welche Methode umfasst:
Vergleichen der Leistung bei Gedächtnis- und Lerntests eines ersten transgenen nicht-humanen Säugers nach Anspruch 20 oder Anspruch 21, der mit dem Agens kontaktiert wurde, und eines zweiten transgenen nicht-humanen Säugers nach Anspruch 20 oder Anspruch 21, der nicht mit dem Agens kontaktiert wurde; und
Vergleichen der Neuropathologie in einer cortico-limbischen Region des Gehirns der ersten und der zweiten transgenen nicht-humanen Säuger, wenn der erste transgene nicht-humane Säuger eine überlegene Leistung bei den Gedächtnisund Lemtests im Vergleich zu dem zweiten transgenen nicht-humanen Säuger erbringt, wobei ein Agens, das zur Behandlung von Alzheimer-Krankheit nützlich ist, durch eine Verminderung der neuropathologischen Befunde im ersten transgenen nicht-humanen Säuger im Vergleich zum zweiten transgenen nicht-humanen Säuger identifiziert wird.

25. Methode zum Screening auf ein Agens, welches zur Behandlung von Alzheimer-Krankheit nützlich ist, welche Methode umfasst:
Vergleichen der Leistung bei Gedächtnis- und Lerntests eines ersten transgenen nicht-humanen Säugers nach Anspruch 20 oder Anspruch 21, der mit dem Agens kontaktiert wurde, und eines zweiten transgenen nicht-humanen Säugers nach Anspruch 20 oder Anspruch 21, der nicht mit dem Agens kontaktiert wurde; und
Vergleichen des Todesalters der ersten und der zweiten transgenen nicht-humanen Säuger, wenn der erste transgene nicht-humane Säuger eine überlegene Leistung bei den Gedächtnis- und Lerntests im Vergleich zu dem zweiten transgenen nicht-humanen Säuger erbringt, wobei ein Agens, das zur Behandlung von Alzheimer-Krankheit nützlich ist, durch ein höheres Todesalter des ersten transgenen nicht-humanen Säugers im Vergleich zum zweiten transgenen nicht-humanen Säuger identifiziert wird.

26. Methode zum Screening auf ein Agens, welches Symptome von Alzheimer-Krankheit mildert, welche Methode umfasst:
Vergleichen des explorativen Verhaltens oder lokomotorischen Verhaltens eines ersten transgenen nicht-humanen Säugers nach Anspruch 20 oder Anspruch 21, der mit dem Agens kontaktiert wurde, und eines zweiten transgenen nichthumanen Säugers nach Anspruch 20 oder Anspruch 21, der nicht mit dem Agens kontaktiert wurde, wobei ein Agens, welches die Symptome mildert, durch ein weniger beeinträchtigtes exploratives oder lokomotorisches Verhalten des ersten transgenen nicht-humanen Säugers im Vergleich zum zweiten trangenen nicht-humanen Säuger identifiziert wird.

## Revendications

1. Procédé pour produire un mammifère transgénique non humain avec une maladie neurologique évolutive dans les zones cortico-limbique du cerveau, ledit procédé comprenant l'incorporation dans un chromosome d'une cassette d'expression qui comprend une séquence codant un précurseur de peptide β-amyloïde jointe de façon opérationnelle à des séquences de régulation, pouvant être obtenues à partir d'un gène de protéine de prion, qui permettent l'expression de ladite séquence codante dans des tissus nerveux à un niveau qui est égal au moins deux à quatre fois celui des niveaux endogènes de précurseur de peptide β-amyloïde de type sauvage dans le génome d'un mammifère non humain.

2. Procédé selon la revendication 1, dans lequel ladite séquence codante est une séquence codante humaine.

3. Procédé selon la revendication 1 ou 2, dans lequel la séquence codante est une séquence codante mutée liée à une maladie.

4. Procédé selon la revendication 3, dans laquelle ladite séquence codante est choisie dans l'ensemble consistant en CS1HuAPP695.V717I.V721A.M722V., CS2HuAPP695.V7171.V721A.M722V., et CS1HuAPP695.K670N.M671L, dans laquelle la séquence d'initiation de la traduction CS1, est GTCGACACCATGC et la séquence d'initiation de la traduction CS2 est GTCGACACCATGG.

5. Procédé selon l'une quelconque des revendications 1 à 3, dans lequel ladite séquence codante est une séquence codante chimérique.

6. Procédé selon la revendication 5, dans lequel ladite séquence codante chimérique est une séquence codante chimérique humaine-murine.

7. Procédé selon l'une quelconque des revendications 1 à 6, dans lequel ladite séquence de régulation comprend une séquence choisie parmi ACCATG, ACCATG, ACCATGG, et ACCATGG, où ATG est le codon d'initiation.

8. Procédé selon l'une quelconque des revendications 1 à 7, dans lequel ladite cassette d'expression comprend une région d'ADN flanquant les séquences de régulation du prion, grâce à quoi on obtient une expression de transgène dépendante du nombre de copies.

9. Procédé selon l'une quelconque des revendications 1 à 8, dans lequel ladite maladie neurologique évolutive est une affection neurocomportementale avec une gliose et une absorption de glucose ou une utilisation de glucose diminuée, ou les deux.

10. Procédé selon l'une quelconque des revendications 1 à 9, dans lequel au moins vingt copies de la cassette d'expression sont incorporées dans le génome.

11. Zygote d'un mammifère non humain comprenant:
une cassette d'expression définie dans l'une quelconque des revendications 1 à 8.

12. Zygote selon la revendication 11, ledit zygote étant dérivé d'un croisement entre rongeurs de la même espèce.

13. Zygote selon la revendication 12, où lesdits rongeurs sont des souris.

14. Zygote selon la revendication 13, où lesdites souris sont des souris FBV.

15. Zygote selon la revendication 13, où lesdites souris sont de souches différentes.

16. Zygote selon la revendication 15, où lesdites souris sont une Swiss Webster et un hybride CS7B16/DBA-ZF1

17. Embryon développé à partir du zygote selon l'une quelconque des revendications 11 à 16.

18. Animal développé à partir de l'embryon selon la revendication 17.

19. Vecteur cosmide comprenant une cassette d'expression telle que définie dans l'une quelconque des revendications 1 à 8.

20. Mammifère transgénique non humain dont les cellules germinales et les cellules somatiques comprennent une cassette d'expression qui fournit un niveau d'expression de précurseur du peptide β-amyloïde mutant qui produit une maladie neurologique évolutive dans les zones corticolimbiques du cerveau dudit mammifère, ladite cassette d'expression étant définie dans l'une quelconque des revendications 1 à 8.

21. Mammifère transgénique non humain tel que revendiqué dans la revendication 20, ledit mammifère présentant une quantité d'expression de précurseur de peptide β-amyloïde mutant dans les tissus nerveux dudit mammifère suffisante pour produire des performances altérées dudit mammifère dans des tests de mémoire et d'apprentissage et pour induire une neuropathologie anormale dans une région cortico-limbique du cerveau dudit mammifère, lesdites performances altérées et ladite neuropathologie anormale étant comparées à celles de mammifères témoins.

22. Descendance dudit mammifère transgénique non humain selon la revendication 20 ou la revendication 21, les cellules germinales et les cellules somatiques de ladite descendance comprenant ladite cassette d'expression.

23. Procédé de criblage d'un agent qui améliore les symptômes de la maladie d'Alzheimer, ledit procédé comprenant :
la comparaison des performances dans des tests de mémoire et d'apprentissage d'un premier mammifère transgénique non humain de la revendication 20 ou de la revendication 21 mis en contact avec ledit agent et d'un second mammifère transgénique non humain de la revendication 20 ou de la revendication 21 qui n'est pas mis en contact avec ledit agent, grâce à laquelle on identifie un agent qui améliore lesdits symptômes par des performances supérieures dudit premier mammifère transgénique non humain par rapport audit second mammifère transgénique non humain dans lesdits tests de mémoire et d'apprentissage.

24. Procédé de criblage d'un agent utile pour le traitement de la maladie d'Alzheimer, ledit procédé comprenant :
la comparaison des performances dans des tests de mémoires et d'apprentissage d'un premier mammifère transgénique non humain de la revendication 20 ou de la revendication 21 mis en contact avec ledit agent et d'un second mammifère transgénique de la revendication 20 ou 21 qui n'est pas mis en contact avec ledit agent ; et
la comparaison de la neuropathologie dans une région cortico-limbique du cerveau desdits premier et second mammifères transgéniques non humains lorsque ledit premier mammifère transgénique non humain présente des performances supérieures dans lesdits tests de mémoire et d'apprentissage par rapport audit second mammifère transgénique non humain, grâce à quoi un agent qui est utile pour le traitement de la maladie d'Alzheimer est identifié par une diminution des résultats neuropathologiques chez ledit premier mammifère transgénique non humain par rapport audit second mammifère transgénique non humain.

25. Procédé de criblage d'un agent utile pour le traitement de la maladie d'Alzheimer, ledit procédé comprenant :
la comparaison des performances dans des tests de mémoire et d'apprentissage d'un premier mammifère transgénique non humain de la revendication 20 ou de la revendication 21 mis en contact avec ledit agent et d'un second mammifère transgénique non humain de la revendication 20 ou 21 qui n'est pas mis en contact avec ledit agent ; et
la comparaison de l'âge au décès desdits premier et second mammifères transgéniques non humains lorsque ledit premier mammifère transgénique non humain présente des performances supérieures dans lesdits tests de mémoire et d'apprentissage par rapport audit second mammifère transgénique non humain, grâce à quoi un agent qui est utile pour le traitement de la maladie d'Alzheimer est identifié par un âge plus élevé au décès du premier mammifère transgénique non humain par rapport audit second mammifère transgénique non humain.

26. Procédé pour cribler un agent qui améliore les symptômes de la maladie d'Alzheimer, ledit procédé comprenant :
la comparaison du comportement exploratoire ou du comportement locomoteur d'un premier mammifère transgénique non humain de la revendication 20 ou de la revendication 21 mis en contact avec ledit agent et d'un second mammifère transgénique non humain de la revendication 20 ou de la revendication 21 qui n'est pas mis en contact avec ledit agent, grâce à quoi un agent qui améliore lesdits symptômes est identifié par un comportement exploratoire ou locomoteur moins altéré chez ledit premier mammifère transgénique non humain par rapport audit second mammifère transgénique non humain.
